# EUROPEAN PATENT SPECIFICATION

(11) **EP 2 316 395 B1**
(45) Date of publication and mention of the grant of the patent: **01.06.2016**
(21) Application number: 10188473.2
(22) Date of filing: 22.10.2010
(51) Int. Cl.: A61F 13/00, A61F 13/40, A61M 25/02, A61F 13/02

(54) **Medical pressure-sensitive adhesive tape**
Druckempfindliches medizinisches Klebeband
Bande adhésive médicale sensible à la pression

(30) Priority: 27.10.2009 JP 2009246140; 02.02.2010 JP 2010020883
(43) Date of publication of application: 04.05.2011
(60) Divisional application: 14190540.6
(73) Proprietor: NITTO DENKO CORPORATION, Ibaraki-shi, Osaka 567-8680 (JP)
(72) Inventor: Hatanaka, Hiroshi, OSAKA 567-8680 (JP); Tsuji, Takashi, OSAKA 567-8680 (JP); Hamada, Atsushi, OSAKA 567-8680 (JP); Yoshikawa, Toshiyuki, OSAKA 567-8680 (JP)
(74) Representative: Augarde, Eric

(56) References cited:
- EP-A2- 0 117 632
- GB-A- 2 297 260
- JP-A- 61 288 860
- JP-A- 2003 153 938
- US-A1- 2004 220 505

## Description

This application claims priority under 35 U.S.C. Section 119 to Japanese Patent Application Nos. 2009-246140 filed on October 27, 2009, and 2010-020883 filed on February 2, 2010, which are herein incorporated by references.

### BACKGROUND OF THE INVENTION

### 1. Field of the Invention

The present invention relates to a medical pressure-sensitive adhesive tape, and more specifically, to a medical pressure-sensitive adhesive tape which is excellent in operability, and can be attached to the surface of a living organism without the generation of wrinkles.

### 2. Description of the Related Art

Medical pressure-sensitive adhesive tapes have been widely used for the protection of affected areas in the surfaces of living organisms (such as a skin), the fixation of gauzes, medical instruments (such as a catheter), and the like to the surfaces of the living organisms, and the transdermal absorption of drugs. In ordinary cases, such medical pressure-sensitive adhesive tapes are provided together with release liners, and the release liners are released before the tapes are applied to the surfaces of the living organisms (forexample, Japanese Utility Model Application Laid-open No. Sho 58-124123, Japanese Patent Application Laid-open No. 2008-295568, and Japanese Patent Application Laid-open No. 2009-136557). The medical pressure-sensitive adhesive tapes after the release of the release liners are thin and have flexibility, and hence wrinkles are apt to be generated. The medical pressure-sensitive adhesive tapes each attached in a state in which wrinkles are generated each involve the following problems. That is, the tapes each have insufficient adhesiveness, a portion floated or released by the wrinkles serves as a route for microbism, and a desired drug absorption cannot be obtained when the transdermal absorption of a drug is targeted. In addition, when one attempts to attach any such tape while preventing the generation of wrinkles, working efficiency deteriorates. In view of the foregoing, a medical pressure-sensitive adhesive tape that can be attached with good operability has been demanded. EP0117632, JP2003153938, JP61288860, US2004220505 and GB2297260 are other examples showing related art.

### SUMMARY OF THE INVENTION

The present invention has been made to solve the above-mentioned conventional problems, and an object of the present invention is to provide a medical pressure-sensitive adhesive tape which is excellent in operability and can be attached to the surface of a living organism without the generation of wrinkles.

The present invention is defined in independent claims 1 and 2, while preferable features are provided in the dependent claims.

### BRIEF DESCRIPTION OF THE DRAWINGS

FIG. **1A** is a schematic plan view of a medical pressure-sensitive adhesive tape according to an example useful for understanding the invention, and FIG. **1B** is a schematic sectional view of the medical pressure-sensitive adhesive tape of FIG. **1A** taken along the line **Ib-Ib.**
FIG. **2A** is a schematic plan view of a medical pressure-sensitive adhesive tape according to another example useful for understanding the invention, and FIG. **2B** is a schematic sectional view of the medical pressure-sensitive adhesive tape of FIG. **2A** taken along the line **IIb**-**IIb**.
FIG. **3** is a schematic sectional view of a medical pressure-sensitive adhesive tape according to a preferred embodiment of the present invention.
FIG. **4** is a schematic sectional view of a medical pressure-sensitive adhesive tape according to another preferred embodiment of the present invention.
FIG. **5A** is a schematic plan view of a medical pressure-sensitive adhesive tape according to still another example useful for understanding the invention viewed from the side opposite to a release liner side, and FIG. **5B** is a schematic sectional view of the medical pressure-sensitive adhesive tape of FIG. **5A** taken along the line **Vb-Vb.**
FIG. **6A** is a schematic plan view of a medical pressure-sensitive adhesive tape according to still another example useful for understanding the invention viewed from the side opposite to a release liner side, and FIG. **6B** is a schematic sectional view of the medical pressure-sensitive adhesive tape of FIG. **6A** taken along the line **VIb-VIb**.
FIG. **7A** is a schematic plan view of a medical pressure-sensitive adhesive tape according to still another example useful for understanding the invention viewed from the side opposite to a release liner side, and FIG. **7B** is a schematic sectional view of the medical pressure-sensitive adhesive tape of FIG. **7A** taken along the line **VIIb-VIIb.**
FIG. **8A** is a schematic plan view of a medical pressure-sensitive adhesive tape according to still another example useful for understanding the invention viewed from the side opposite to a release liner side, and FIG. **8B** is a schematic sectional view of the medical pressure-sensitive adhesive tape of FIG. **8A** taken along the line **VIIIb-VIIIb.**
FIG. **9** is an exploded perspective diagram illustrating a medical pressure-sensitive adhesive tape according to still another preferred embodiment of the present invention.
FIG. **10A** is a schematic plan view of a medical pressure-sensitive adhesive tape according to an example useful for understanding the invention, and FIG. **10B** is a schematic sectional view of the medical pressure-sensitive adhesive tape of FIG. **10A** taken along the line **Xb-Xb**.
FIG. **11** is a schematic sectional view of a medical pressure-sensitive adhesive tape according to still another example useful for understanding the invention in the case where a cut portion is provided and a release liner is divided into two pieces.
FIG. **12** is a schematic rear surface view of a medical pressure-sensitive adhesive tape according to still another example useful for understanding the invention.
FIG. **13** is a schematic sectional view of a medical pressure-sensitive adhesive tape according to still another example useful for understanding the invention.
FIG. **14** is a schematic sectional view of a medical pressure-sensitive adhesive tape according to still another example useful for understanding the invention.
FIG. **15A** is a view illustrating the notches in a state in which the release liner is folded back, and FIG. **15B** is a view illustrating the notches in a state in which the release liner is unfolded. FIG. **15C** is a view illustrating the one divided piece retains the other divided piece with the notches. Figures 15A to 15C show examples useful for understanding the invention.
FIGS. **16A** to **16D** are schematic diagrams for illustrating a specific attachment procedure for the medical pressure-sensitive adhesive tape of the embodiment illustrated in FIG. **4**.
FIGS. **17A**to **17D** are schematic diagrams for illustrating a specific attachment procedure for the medical pressure-sensitive adhesive tape of the embodiment illustrated in FIG. **9**.
FIGS. **18A** to **18G** are schematic diagrams for illustrating a specific attachment procedure for a medical pressure-sensitive adhesive tape in the case where a cut portion is provided and a release liner is divided into two pieces according to an example useful for understanding the invention.
FIGS. **19A** to **19F** are schematic diagrams for illustrating a specific attachment procedure for a medical pressure-sensitive adhesive tape (representatively a film dressing) in the case where a cut portion is provided and a release liner is divided into three pieces according to an example useful for understanding the invention.
FIG. **20** is a schematic perspective diagram for illustrating a state in which the main body of the medical pressure-sensitive adhesive tape illustrated in each of FIGS. **10** is attached to the surface of a living organism according to an example useful for understanding the invention.

### DETAILED DESCRIPTION OF THE PREFERRED EMBODIMENTS

Hereinafter, preferred embodiments of the present invention are described with reference to drawings. However, the present invention is not limited to these specific embodiments. Examples useful for understanding the invention are also hereinafter described with reference to drawings.

### A. Entire constitution of medical pressure-sensitive adhesive tape

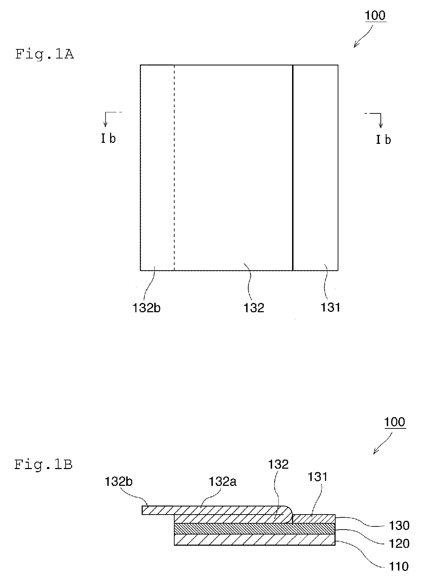

FIG. **1A** is a schematic plan view of a medical pressure-sensitive adhesive tape according to an example useful for understanding the invention, and FIG. **1B** is a schematic sectional view of the medical pressure-sensitive adhesive tape of FIG. **1A** taken along the line **Ib-Ib**. It should be noted that, for ease of viewing, each layer and each part in the illustrated example are illustrated while a ratio among the length, width, and thickness of each of them is different from an actualone. A medical pressure-sensitive adhesive tape **100** includes a film base material **110**, a first pressure-sensitive adhesive layer **120**, and a release liner **130** releasably laminated to cover the first pressure-sensitive adhesive layer **120** in the stated order. It should be noted that FIG. **1A** is a schematic plan view when the medical pressure-sensitive adhesive tape **100** is viewed from the side of the release liner **130**. In addition, in the specification, a laminate excluding the release liner (a laminate of the film base material **110** and the first pressure-sensitive adhesive layer **120** in each of FIGS. **1**) may be called the main body of the medical pressure-sensitive adhesive tape. The planar-view shape of the medical pressure-sensitive adhesive tape is not limited to the illustrated example, and needless to say, any appropriate shape (such as a circle, a square, a rectangle, or a trapezoid) can be adopted depending on purposes. When a square or a rectangle is adopted as the planar-view shape, the medical pressure-sensitive adhesive tape can be provided in a roll shape wound in its lengthwise direction (that is, the vertical direction of the space of FIG. **1A** or the normal direction of the space of FIG. **1B**), and can be used after having been cut into any appropriate length as required.

The release liner **130** is divided into a first divided piece **131** and a second divided piece **132**. The entirety of the first pressure-sensitive adhesive layer **120** is covered with the first divided piece **131** and the second divided piece **132**. The first divided piece **131** is placed at a widthwise-direction end on one side of the first pressure-sensitive adhesive layer **120** (that is, an end on the right side of the space of each of FIGS. **1A** and **1B**) so as to cover part of the first pressure-sensitive adhesive layer **120**. The second divided piece **132** is placed so as to cover the portion of the first pressure-sensitive adhesive layer **120** not covered with the first divided piece **131**. When the release liner **130** is divided as described above, the attachment of the main body of the medical pressure-sensitive adhesive tape to a desired position can be completed with good operability and without the generation of wrinkles because of the following reason. That is, the first divided piece **131** is released first, and then the portion of the first pressure-sensitive adhesive layer **120** corresponding to the first divided piece **131** is attached to the surface of a living organism (such as a skin) so that the main body of the medical pressure-sensitive adhesive tape may be temporally stacked. After that, the second divided piece **132** is released, and hence the portion of the first pressure-sensitive adhesive layer **120** corresponding to the second divided piece **132** can be attached to the surface of the living organism. In addition, upon attachment of the portion of the medical pressure-sensitive adhesive tape **100** of the present example corresponding to the first divided piece **131**, the attachment can be performed by holding the second divided piece **132**. In addition, upon release of the second divided piece **132**, the portion corresponding to the first divided piece **131** is in a state of being attached to the surface of the living organism. Accordingly, attachment can be performed without any touch to the first pressure-sensitive adhesive layer **120**.

The width of the first pressure-sensitive adhesive layer **120** covered with the first divided piece **131** (that is, the width of the portion of the first pressure-sensitive adhesive layer **120** in direct contact with the first divided piece **131**) accounts for preferably 10% to 80%, more preferably 20% to 60%, or particularly preferably 20% to 40% of the total width of the first pressure-sensitive adhesive layer **120**.

The second divided piece **132** has a folding portion **132a** extending toward a widthwise-direction end. The folding portion **132a** of the second divided piece preferably has an extending portion **132b** of the second divided piece extending toward the outside of the film base material **110**. With such constitution, the operability with which the second divided piece **132** is released from the main body of the medical pressure-sensitive adhesive tape that has been temporally stacked can be markedly improved. As a result, the main body of the medical pressure-sensitive adhesive tape can be caused to adhere to the surface of the living organism favorably by a simple operation.

The first divided piece **131** preferably has a fold or perforation (not shown) substantially parallel to the side at the widthwise-direction end. When the first divided piece **131** has the fold or perforation, the operability with which the first divided piece is released is improved. In addition, at the time of the temporal stacking, the first divided piece **131** is folded back from the fold or perforation so that part of the first divided piece **131** may be released from the first pressure-sensitive adhesive layer **120**. The first pressure-sensitive adhesive layer **120** thus exposed is attached to the surface of the living organism. After that, the entirety of the first divided piece **131** can be released. Therefore, when the first divided piece **131** has the fold or perforation, the first divided piece **131** can be released in a stepwise manner. Accordingly, operability at the time of each of the release of the first divided piece **131** and the temporal stacking of the main body of the medical pressure-sensitive adhesive tape is improved.

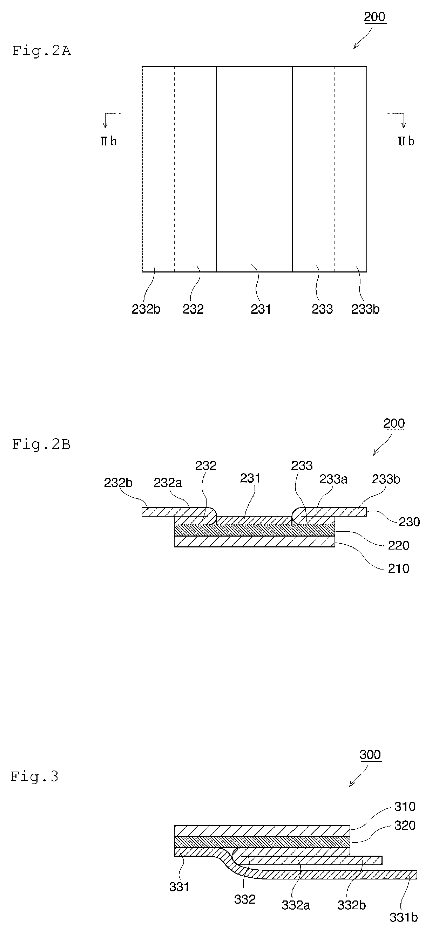

FIG. **2A** is a schematic plan view of a medical pressure-sensitive adhesive tape according to another an example useful for understanding the invention, and FIG. **2B** is a schematic sectional view of the medical pressure-sensitive adhesive tape of FIG. **2A** taken along the line **IIb-IIb**. A release liner **230** of a medical pressure-sensitive adhesive tape **200** is divided into a first divided piece **231**, a second divided piece **232**, and a third divided piece **233**. The entirety of a first pressure-sensitive adhesive layer **220** is covered with the first divided piece **231**, the second divided piece **232**, and the third divided piece **233**. The second divided piece **232** is placed at a widthwise-direction end on one side of the first pressure-sensitive adhesive layer **220** (that is, an end on the left side of the space of each of FIGS. **2A** and **2B**) so as to cover part of the first pressure-sensitive adhesive layer **220**. The third divided piece **233** is placed at a widthwise-direction end on the other side of the first pressure-sensitive adhesive layer **220** (that is, an end on the right side of the space of each of FIGS. **2A** and **2B**) so as to cover part of the first pressure-sensitive adhesive layer **220**. The first divided piece **231** is placed between the second divided piece **232** and the third divided piece **233** in a widthwise direction so as to cover the portion of the first pressure-sensitive adhesive layer **220** not covered with the second divided piece **232** and the third divided piece **233**. When the release liner **230** is divided as described above, the attachment of the main body of the medical pressure-sensitive adhesive tape to a desired position can be completed with good operability and without the generation of wrinkles because of the following reason. That is, the first divided piece **231** is released first, and then the portion of the first pressure-sensitive adhesive layer **220** corresponding to the first divided piece **231** is attached to the surface of a living organism (such as a skin) so that the main body of the medical pressure-sensitive adhesive tape may be temporally stacked. After that, the second divided piece **232** and the third divided piece **233** are released, and hence the portions of the first pressure-sensitive adhesive layer **220** corresponding to the second divided piece **232** and the third divided piece **233** can be attached to the surface of the living organism. In addition, upon attachment of the portion of the medical pressure-sensitive adhesive tape **200** of the present example corresponding to the first divided piece **231**, the attachment can be performed by holding at least one of the second divided piece **232** and the third divided piece **233**. In addition, upon release of the second divided piece **232** and the third divided piece **233**, the portion corresponding to the first divided piece **231** is in a state of being attached to the surface of the living organism. Accordingly, attachment can be performed without any touch to the first pressure-sensitive adhesive layer **220**.

The width of each of the first divided piece **231,** the second divided piece **232**, and the third divided piece **233** can be set to any appropriate width. The width of the first pressure-sensitive adhesive layer **220** covered with the first divided piece **231** (that is, the width of the portion of the first pressure-sensitive adhesive layer **220** in direct contact with the first divided piece **231**) accounts for preferably 10% to 80%, more preferably 20% to 60%, or particularly preferably 20% to 40% of the total width of the first pressure-sensitive adhesive layer **220**. In addition, the width of the first pressure-sensitive adhesive layer **220** covered with the second divided piece **232** and the width of the first pressure-sensitive adhesive layer **220** covered with the third divided piece **233** may be equal to or different from each other.

The second divided piece **232** and the third divided piece **233** have folding portions **232a** and **233a** extending toward the widthwise-direction ends, respectively. The folding portion **232a** of the second divided piece preferably has an extending portion **232b** of the second divided piece extending toward the outside of a film base material **210**, and the folding portion **233a** of the third divided piece preferably has an extending portion **233b** of the third divided piece extending toward the outside of the film base material **210**. With such constitution, the operability with which the second divided piece **232** and the third divided piece **233** are released from the main body of the medical pressure-sensitive adhesive tape that has been temporally stacked can be markedly improved. As a result, the medical pressure-sensitive adhesive tape can be caused to adhere to the surface of the living organism favorably by a simple operation.

FIG. **3** is a schematic sectional view of a medical pressure-sensitive adhesive tape according to a preferred embodiment of the present invention. FIG. **3** illustrates a medical pressure-sensitive adhesive tape **300** where the first divided piece in each of FIGS. **1** is placed so as to further cover at least part of the second divided piece. In the medical pressure-sensitive adhesive tape **300**, a first divided piece **331** is placed on the side of a first pressure-sensitive adhesive layer **320** opposite to a film base material **310** and the side of a second divided piece **332** opposite to the first pressure-sensitive adhesive layer **320** so as to cover the portion of the first pressure-sensitive adhesive layer **320** not covered with the second divided piece **332** and at least part of the second divided piece **332**. With such constitution, the operability with which the first divided piece **331** is released from the main body of the medical pressure-sensitive adhesive tape can be improved.

The first divided piece **331** has an extending portion **331b** of the first divided piece extending toward the outside in the widthwise direction of the film base material **310**. The extending portion **331b** of the first divided piece extends toward the outside of the second divided piece **332**. Therefore, as illustrated in FIG. **3**, the second divided piece **332** is provided with a folding portion **332a** and an extending portion **332b** extending over and beyond the film base material, and the extending portion **331b** of the first divided piece extends beyond the extending portion **332b** of the second divided piece. With such constitution, the extending portion **331b** of the first divided piece to be released first at the time of the attachment of the medical pressure-sensitive adhesive tape extends toward the outermost side. Accordingly, the extending portion **331b** of the first divided piece can function as a holding margin in an additionally effective fashion, and the operability with which the first divided piece **331** is released from the main body of the medical pressure-sensitive adhesive tape can be markedly improved. Although not illustrated, the first divided piece **331** may have extending portions on both sides in the widthwise direction (that is, the piece may further have an extending portion on the left side of the space of FIG. **3**).

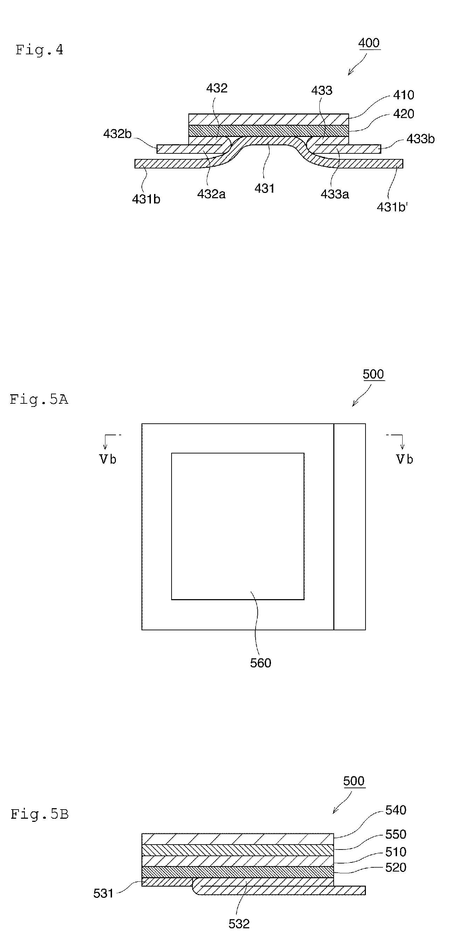

FIG. **4** is a schematic sectional view of a medical pressure-sensitive adhesive tape according to another preferred embodiment of the present invention. FIG. **4** illustrates a medical pressure-sensitive adhesive tape **400** where the first divided piece in each of FIGS. **2** is placed so as to further cover at least part of the second divided piece and the third divided piece. In the medical pressure-sensitive adhesive tape **400**, a first divided piece **431** is placed on the side of a first pressure-sensitive adhesive layer **420** opposite to a film base material **410** and between a second divided piece **432** and a third divided piece **433** opposite to the first pressure-sensitive adhesive layer **420** so as to cover the portion of the first pressure-sensitive adhesive layer **420** not covered with the second divided piece **432** and the third divided piece **433**, and at least part of the second divided piece **432** and the third divided piece **433**. With such constitution, the operability with which the first divided piece **431** is released from the main body of the medical pressure-sensitive adhesive tape can be improved.

The first divided piece **431** has extending portions **431b** and **431b**' of the first divided piece each extending toward the outside in the widthwise direction of the film base material **410**. The extending portion **431b** and **431b**' of the first divided piece extend toward the outside of the second divided piece **432** and the third divided piece **433**. Therefore, as illustrated in FIG. **4**, the second divided piece **432** is provided with a folding portion **432a** and an extending portion **432b**, the third divided piece **433** is provided with a folding portion **433a** and an extending portion **433b** extending over and beyond the film base material, and the extending portions **431b** and **431b**' of the first divided piece extend beyond the extending portion **432b** of the second divided piece and the extending portion **433b** of the third divided piece. With such constitution, the extending portions **431b** and **431b**' of the first divided piece to be released first at the time of the attachment of the medical pressure-sensitive adhesive tape each extend toward the outermost side. Accordingly, each of the extending portions **431b** and **431b**' of the first divided piece can function as a holding margin in an additionally effective fashion, and the operability with which the first divided piece **431** is released from the main body of the medical pressure-sensitive adhesive tape can be markedly improved.

FIG. **5A** is a schematic plan view of a medical pressure-sensitive adhesive tape according to still another example useful for understanding the invention viewed from the side opposite to a release liner side, and FIG. **5B** is a schematic sectional view of the medical pressure-sensitive adhesive tape of FIG. **5A** taken along the line **Vb-Vb**. A medical pressure-sensitive adhesive tape **500** illustrated in each of the figures further includes a support **540** in addition to the constitution illustrated in each of FIGS. **1**. The support **540** is placed on the side of a film base material **510** opposite to a first pressure-sensitive adhesive layer **520** through a second pressure-sensitive adhesive layer **550**. When the medical pressure-sensitive adhesive tape **500** includes the support **540**, rigidity and shape retentivity are imparted to the main body of the medical pressure-sensitive adhesive tape, and its handleability can be improved. The support **540** is preferably provided with a window portion **560**. When the support **540** has the window portion **560**, in, for example, the case where the medical pressure-sensitive adhesive tape covers an affected area in the surface of a living organism or covers the puncture portion of a medical instrument, the medical pressure-sensitive adhesive tape can be attached while the affected area or the puncture portion is observed.

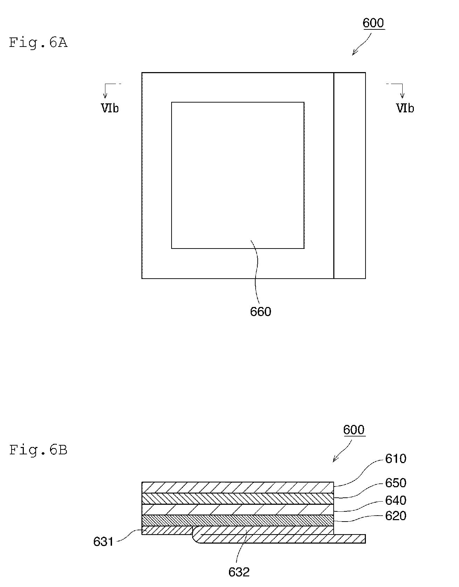

FIG. **6A** is a schematic plan view of a medical pressure-sensitive adhesive tape according to still another example useful for understanding the invention viewed from the side opposite to a release liner side, and FIG. **6B** is a schematic sectional view of the medical pressure-sensitive adhesive tape of FIG. **6A** taken along the line **VIb-VIb**. A medical pressure-sensitive adhesive tape **600** illustrated in each of the figures is such that a support **640** is placed between a film base material **610** and a first pressure-sensitive adhesive layer **620**, and a second pressure-sensitive adhesive layer **650** is placed between the film base material **610** and the support **640**.

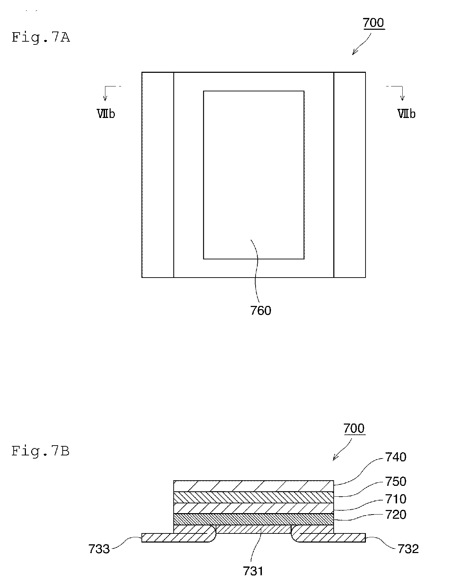

FIG. **7A** is a schematic plan view of a medical pressure-sensitive adhesive tape according to still another example useful for understanding the invention viewed from the side opposite to a release liner side, and FIG. **7B** is a schematic sectional view of the medical pressure-sensitive adhesive tape of FIG. **7A** taken along the line **VIIb-VIIb.** A medical pressure-sensitive adhesive tape **700** illustrated in each of the figures further includes a support **740** in addition to the constitution illustrated in each of FIGS. **2**. The support **740** is placed on the side of a film base material **710** opposite to a first pressure-sensitive adhesive layer **720** through a second pressure-sensitive adhesive layer **750**. The support **740** is preferably provided with a window portion **760**.

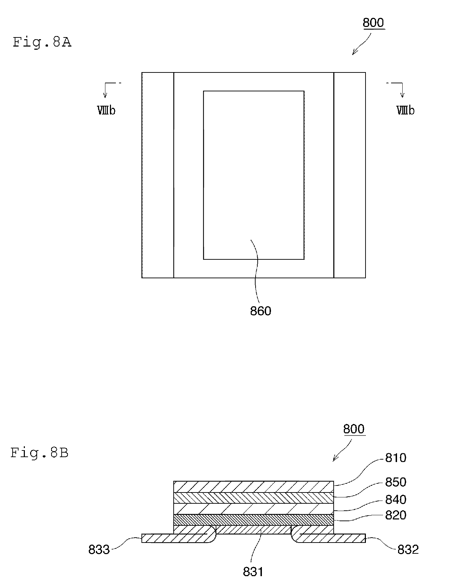

FIG. **8A** is a schematic plan view of a medical pressure-sensitive adhesive tape according to still another example useful for understanding the invention viewed from the side opposite to a release liner side, and FIG. **8B** is a schematic sectional view of the medical pressure-sensitive adhesive tape of FIG. **8A** taken along the line **VIIIb-VIIIb**. A medical pressure-sensitive adhesive tape **800** illustrated in each of the figures is such that a support **840** is placed between a film base material **810** and a first pressure-sensitive adhesive layer **820**, and a second pressure-sensitive adhesive layer **850** is placed between the film base material **810** and the support **840**.

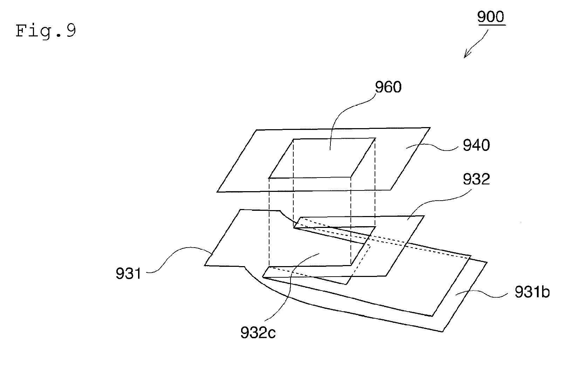

FIG. **9** is an exploded perspective diagram illustrating a medical pressure-sensitive adhesive tape according to still another preferred embodiment of the present invention. FIG. **9** illustrates a medical pressure-sensitive adhesive tape **900** where the first divided piece in each of FIGS. **5** further covers the second divided piece, and has an extending portion extending toward the outside of the second divided piece. The medical pressure-sensitive adhesive tape **900** is such that a support **940** has a window portion **960**, and a second divided piece **932** has a window portion **932c**. It should be noted that the illustration of a film base material, a first pressure-sensitive adhesive layer, and a second pressure-sensitive adhesive layer is omitted in FIG. **9** for understandability of description. In the medical pressure-sensitive adhesive tape **900**, the window portion **932c** of the second divided piece **932** is provided at a position corresponding to the window portion **960** of the support, and the second divided piece **932** does not cover the portion of the first pressure-sensitive adhesive layer corresponding to the window portion **960**. That is, in this embodiment, the second divided piece **932** is placed so as to cover only the portion of the first pressure-sensitive adhesive layer corresponding to the frame portion of the support **940**. In addition, a folding portion **932a** of the second divided piece **932** does not cover the portion of the first pressure-sensitive adhesive layer corresponding to the window portion **960** either. According to such embodiment, a first divided piece **931** is placed so as to cover at least the portion of the first pressure-sensitive adhesive layer that is not covered with the second divided piece. It should be noted that, when a release liner is divided into three pieces, i.e., a first divided piece, a second divided piece, and a third divided piece, the second divided piece and the third divided piece (and the folding portions of these pieces) can be placed so as not to cover the portion of the first pressure-sensitive adhesive layer corresponding to the window portion, and the first divided piece can be placed so as to cover at least the portion of the first pressure-sensitive adhesive layer that is not covered with the second divided piece and the third divided piece. With such constitution, in the case where the medical pressure-sensitive adhesive tape covers an affected area in the surface of a living organism, the medical pressure-sensitive adhesive tape can be attached while the affected area is observed. In addition, in the case where the medical pressure-sensitive adhesive tape covers the puncture portion of a medical instrument, the location of the attachment position of the medical pressure-sensitive adhesive tape and a removal operation for the second divided piece are facilitated, and the medical pressure-sensitive adhesive tape can be attached while the puncture portion is observed. Further, a balance between rigidity and flexibility in a state in which the first divided piece is released is excellent, and the main body of the medical pressure-sensitive adhesive tape can be easily attached even to the surface of a living organism with unevenness.

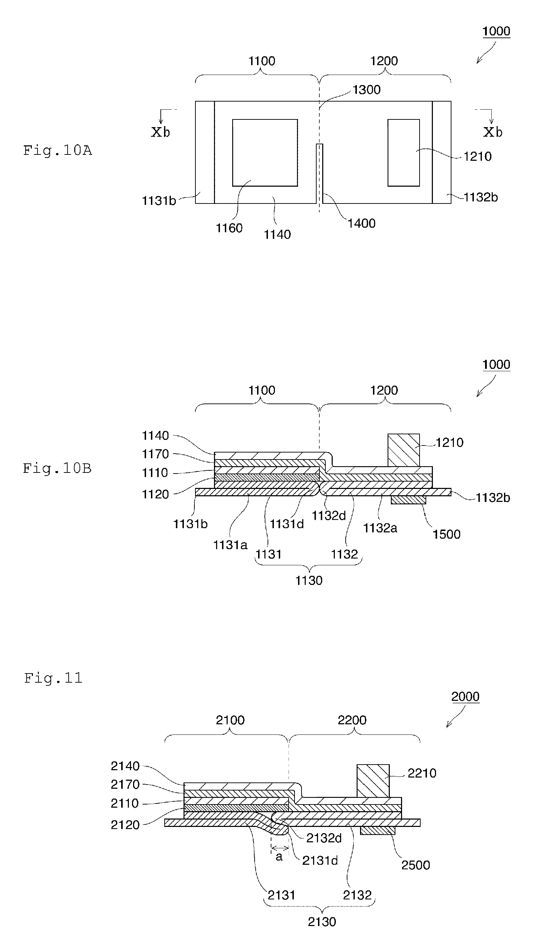

In one example useful for understanding the invention, the medical pressure-sensitive adhesive tape is provided with a cut portion. The medical pressure-sensitive adhesive tape provided with the cut portion as described above can be suitably used as a film dressing capable of favorably fixing a medical instrument such as a catheter at its puncture portion. FIG. **10A** is a schematic plan view of a medical pressure-sensitive adhesive tape according to an example useful for understanding the invention, and FIG. **10B** is a schematic sectional view of the medical pressure-sensitive adhesive tape of FIG. **10A** taken along the line **Xb-Xb**. It should be noted that, for ease of viewing, each layer and each part in the illustrated example are illustrated while a ratio among the length, width, and thickness of each of them is different from an actual one. A medical pressure-sensitive adhesive tape **1000** is provided with a cut portion **1400** extending from a lengthwise-direction end (that is, an end on the lower side of the space of FIG. **10A**) in the lengthwise direction (that is, the upward direction of the space of FIG. **10A**). In the medical pressure-sensitive adhesive tape **1000**, a film portion **1100** and a fixation aid portion **1200** are defined by a boundary **1300** substantially parallel to the cut portion **1400**. In the medical pressure-sensitive adhesive tape **1000**, a support **1140**, a third pressure-sensitive adhesive layer **1170**, and a release liner **1130** are provided in the stated order for the film portion **1100** and the fixation aid portion **1200**. In addition, a film base material **1110** and a first pressure-sensitive adhesive layer **1120** are provided for the film portion **1100**. That is, the film portion **1100** and the fixation aid portion **1200** have the common support **1140**, the common third pressure-sensitive adhesive layer **1170**, and the release liner **1130**. The above-mentioned film portion **1100** further has the film base material **1110** and the first pressure-sensitive adhesive layer **1120**. The film base material **1110** is placed between the third pressure-sensitive adhesive layer **1170** and the release liner **1130**, and the first pressure-sensitive adhesive layer **1120** is placed between the film base material **1110** and the release liner **1130**. The fixation aidportion **1200** preferably further has a fixing material **1210** on the side of the support **1140** opposite to the third pressure-sensitive adhesive layer **1170**. The release liner **1130** is releasably laminated to cover the surface of the first pressure-sensitive adhesive layer **1120** in the film portion **1100** and the surface of the third pressure-sensitive adhesive layer **1170** in the fixation aid portion **1200**. The support **1140 of** the filmportion **1100** is provided with a window portion **1160**. The medical pressure-sensitive adhesive tape of the present example can preferably further include a fixing tape **1500**. It is preferred that the fixing tape **1500** be releasably laminated on the portion of the release liner **1130** out of contact with the third pressure-sensitive adhesive layer **1170** or the first pressure-sensitive adhesive layer **1120**. Although not illustrated, the support **1140** and the fixing material **1210**, and the release liner **1130** and the fixing tape **1500** are laminated through any appropriate adhesive or pressure-sensitive adhesive.

The medical pressure-sensitive adhesive tape of this example is excellent in operability and can favorably fix a medical instrument such as a catheter at its puncture portion because of the following reason. That is, the tape is provided with the cut portion **1400**, and hence a bonding portion between the transfusion tube and puncture portion of the medical instrument such as a catheter can be fixed on the above-mentioned fixation aid portion (that is, on the above-mentioned fixing material), and the puncture portion of the medical instrument such as a catheter can be covered with the film base material of the film portion. For example, when such medical pressure-sensitive adhesive tape is used as a film dressing to fix a catheter, such a problem as described below can be prevented. That is, a drug solution leaks to the outside of a blood vessel owing to the movement of the indwelling needle of the catheter, the indwelling needle risks being extracted, or the medical pressure-sensitive adhesive tape is floated or released by the movement of the catheter and the floated or released portion serves as a route for microbism. The cut portion **1400** preferably extends from the lengthwise-direction end of the medical pressure-sensitive adhesive tape along the boundary **1300** between the above-mentioned film portion **1100** and the above-mentioned fixation aid portion **1200** as illustrated in FIG. **10A**. Any appropriate shape can be adopted as the shape of the above-mentioned cut portion as long as an effect of the present example is obtained. Examples of the shape of the above-mentioned cut portion include a linear shape, a wave shape, an arc shape, and a combination of two or more of them. The length of the above-mentioned cut portion accounts for preferably 20% to 90%, more preferably 30% to 90%, or particularly preferably 50% to 70% of the total length of the boundary **1300** between the above-mentioned film portion and the above-mentioned fixation aid portion (that is, the length of the medical pressure-sensitive adhesive tape **1000**). When the length of the above-mentioned cut portion falls within such range, a medical instrument such as a catheter can be favorably fixed. In addition, upon release of the release liner, the medical pressure-sensitive adhesive tape is hardly distorted, and can be prevented from wrinkling and folding. The shortest length from the above-mentioned cut portion to the end of the above-mentioned fixing material on the side of the above-mentioned cut portion is preferably 5 mm to 50 mm, more preferably 10 mm to 35 mm, or particularly preferably 14 mm to 30 mm. When the shortest length from the above-mentioned cut portion to the end of the above-mentioned fixing material on the side of the above-mentioned cut portion is shorter than 5 mm, it may become difficult to cover the puncture portion of a medical instrument such as a catheter with the above-mentioned film portion. When the shortest length is longer than 50 mm, it may be impossible to fix the medical instrument such as a catheter favorably.

The above-mentioned release liner **1130** is preferably divided. A position at which the above-mentioned release liner is divided can be set to any appropriate position as long as the effect of the present example is obtained. The position is preferably a boundary between the film portion and the fixation aid portion, or a vicinity of the boundary. In one example, as illustrated in FIG. **10B**, the above-mentioned release liner **1130** is divided into two pieces, i.e., a first divided piece **1131** and a second divided piece **1132** along the boundary **1300** between the film portion **1100** and the fixation aid portion **1200**. The first divided piece **1131** is releasably laminated to cover the surface of the first pressure-sensitive adhesive layer **1120** in the film portion **1100**. The second divided piece **1132** is releasably laminated to cover the surface of the third pressure-sensitive adhesive layer **1170** in the fixation aid portion **1200**.

The first divided piece **1131** and the second divided piece **1132** described above preferably have folding portions. When the above-mentioned release liner has folding portions, one can easily release the release liner without touching any pressure-sensitive adhesive layer. As a result, excellent operability can be obtained upon attachment of the medical pressure-sensitive adhesive tape to the surface of a living organism (such as a skin). In one example , as illustrated in FIG. **10B****,** the first divided piece **1131** and the second divided piece **1132** have folding portions **1131a** and **1132a** folded back by providing folding lines **1131d** and **1132d** substantially parallel to the boundary **1300** between the film portion **1100** and the fixation aid portion **1200**, respectively in the boundary **1300.** The directions in which the first divided piece **1131** and the second divided piece **1132** are released are directions substantially perpendicular to the folding lines **1131d** and **1132d**. It should be noted that, although the folding line portions are illustrated with curves for convenience in the schematic sectional view, the release liner is actually folded back at an acute angle at each folding line portion. When the first divided piece **1131** and the second divided piece **1132** have the folding portions **1131a** and **1132a**, respectively as described above, the fixing tape **1500** can be placed on the side of one of the folding portions opposite to the pressure-sensitive adhesive layer.

The above-mentioned folding portions **1131a** and **1132a** preferably have extending portions. When the folding portions have extending portions, the extending portions serve as gripping portions, and hence a medical pressure-sensitive adhesive tape significantly excellent in operability upon attachment to the surface of a living organism can be obtained. In one example, as illustrated in FIGS. **10A** and **10B****,** an extending portion **1131b** is provided at the end on the side of the folding portion **1131a** opposite to the folding line **1131d,** and an extending portion **1132b** is provided at the end on the side of the folding portion **1132a** opposite to the folding line **1132d.** The planar-view shape of each of the above-mentioned extending portions is not limited to the illustrated example, and any appropriate planar-view shape can be adopted depending on purposes. Specific examples of the planar-view shape of each of the above-mentioned extending portions include quadrangles (including a trapezoid) and a semi-elliptical shape. In addition, the ends of the above-mentioned extending portions may each be of a wave shape. The above-mentioned extending portions each have a width of preferably 1 mm to 30 mm, or more preferably 5 mm to 20 mm. When the width of each of the above-mentioned extending portions falls within such range, a medical pressure-sensitive adhesive tape having excellent releasing operability and an appropriate size can be obtained.

FIG. **11** is a schematic sectional view of a medical pressure-sensitive adhesive tape according to still another example useful for understanding the invention in the case where a cut portion is provided and a release liner is divided into two pieces. In a medical pressure-sensitive adhesive tape **2000** illustrated in the figure, the end on the folding line side of a first divided piece **2131** and the end on the folding line side of a second divided piece **2132** overlap each other. Further, one of a folding line **2131d** of the first divided piece **2131** and a folding line **2132d** of the second divided piece **2132** is preferably constituted so as to be positioned directly below the above-mentioned cut portion (in the illustrated example, the folding line **2131d** of the first divided piece **2131** is positioned directly below the cut portion). An overlapping width **a** between the first divided piece **2131** and the second divided piece **2132** is preferably 2 mm to 6 mm, or more preferably 3 mm to 5 mm.

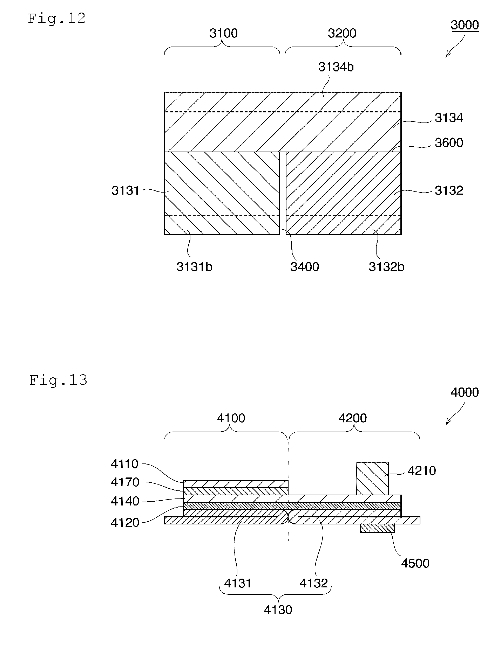

FIG. **12** is a schematic rear surface view of a medical pressure-sensitive adhesive tape according to still another example useful for understanding the invention. In a medical pressure-sensitive adhesive tape **3000** illustrated in the figure, a release liner divided into three pieces is used instead of the release liner divided into two pieces of the example illustrated in each of FIGS. **10**. That is, the release liner is divided into a first divided piece **3131**, a second divided piece **3132**, and a fourth divided piece **3134**. The first divided piece **3131** and the second divided piece **3132** are divided from each other by a cut portion **3400**. A set of the first divided piece **3131** and the second divided piece **3132**, and the fourth divided piece **3134** are divided from each other along a line **3600** extending from the end of the cut portion **3400** in the direction perpendicular to the above-mentioned cut portion **3400**. The first divided piece **3131** is releasably laminated to cover part of the surface of a first pressure-sensitive adhesive layer. The second divided piece **3132** is releasably laminated to cover part of the surface of a third pressure-sensitive adhesive layer in a fixation aid portion **3200**. The fourth divided piece **3134** is releasably laminated to cover the surface of the first pressure-sensitive adhesive layer that is not covered with the first divided piece **3131** in the film portion **3100** and the surface of the third pressure-sensitive adhesive layer that is not covered with the second divided piece **3132** in the fixation aid portion **3200**. Further, the first divided piece **3131**, the second divided piece **3132**, and the fourth divided piece **3134** have folding portions folded back by providing folding lines along the line **3600** extending in the direction perpendicular to the cut portion **3400**. It should be noted that the directions in which the first divided piece **3131**, the second divided piece **3132**, and the fourth divided piece **3134** are released are directions substantially perpendicular to the folding lines. When the release liner is divided into three pieces as described above, excellent operability can be achieved because of the following reason. That is, upon fixation of a medical instrument such as a catheter, the attachment of the portion of the medical pressure-sensitive adhesive tape corresponding to the fourth divided piece **3134** to the surface of a living organism is performed as a first operation so that the medical pressure-sensitive adhesive tape may be aligned, and thereafter, any subsequent attachment operation can be performed. The folding portions are preferably provided with extending portions **3131b, 3132b,** and **3134b** extending toward the outsides of the first pressure-sensitive adhesive layer and the third pressure-sensitive adhesive layer at the ends opposite to the folding lines. When the release liner is divided into three pieces as described above, a fixing tape is placed, for example, on the side of the fourth divided piece **3134** opposite to a pressure-sensitive adhesive layer, though the fixing tape is not illustrated.

FIG. **13** is a schematic sectional view of a medical pressure-sensitive adhesive tape according to still another example useful for understanding the invention. A medical pressure-sensitive adhesive tape **4000** illustrated in the figure is different from the medical pressure-sensitive adhesive tape illustrated in each of FIGS . **10** in the placement of each of a support, a third pressure-sensitive adhesive layer, a film base material, and a first pressure-sensitive adhesive layer. In the medical pressure-sensitive adhesive tape **4000,** a support **4140,** a first pressure-sensitive adhesive layer **4120,** and a release liner **4130** are provided in the stated order for a filmportion **4100** and a fixation aid portion **4200.** In addition, a film base material **4110** and a third pressure-sensitive adhesive layer **4170** are provided for the film portion **4100.** The film base material **4110** is placed on the side of the support **4140** opposite to the first pressure-sensitive adhesive layer **4120** in the film portion **4100.** The third pressure-sensitive adhesive layer **4170** is placed between the film base material **4110** and the support **4140.**

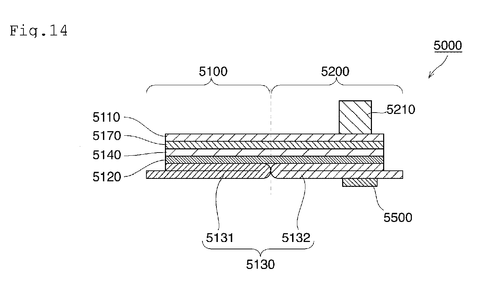

FIG. **14** is a schematic sectional view of a medical pressure-sensitive adhesive tape according to still another example useful for understanding the invention. In a medical pressure-sensitive adhesive tape **5000** illustrated in the figure, a film base material **5110,** a third pressure-sensitive adhesive layer **5170,** a support **5140,** a first pressure-sensitive adhesive layer **5120**, and a release liner **5130** are provided in the stated order for a film portion **5100** and a fixation aid portion **5200**. In addition, a fixing material **5210** is provided on the side of the film base material **5110** opposite to the third pressure-sensitive adhesive layer **5170.** That is, in the film dressing **5000,** the film portion **5100** and the fixation aid portion **5200** have the common film base material **5110,** the common third pressure-sensitive adhesive layer **5170,** the common support **5140,** the common first pressure-sensitive adhesive layer **5120,** and the release liner **5130.** In the film portion **5100,** the film base material **5110** is placed on the side of the support **5140** opposite to the first pressure-sensitive adhesive layer **5120,** and the third pressure-sensitive adhesive layer **5170** is placed between the film base material **5110** and the support **5140.** In addition, in the fixation aid portion **5200**, the film base material **5110** is placed between the fixing material **5210** and the support **5140**, and the third pressure-sensitive adhesive layer **5170** is placed between the film base material **5110** and the support **5140**. Although not illustrated, the film base material **5110** and the fixing material **5210** are attached to each other with any appropriate adhesive or pressure-sensitive adhesive.

Two or more of the embodiments of the medical pressure-sensitive adhesive tape of the present invention described above can be appropriately combined as long as the effect of the present invention is obtained.

### B. Description of each component

### B-1. Film base material

Any appropriate film can be adopted as the above-mentioned film base material as long as the film can be used in the medical pressure-sensitive adhesive tape. A film having flexibility and doing no harm to the surface of a living organism such as a skin can be representatively adopted. A material for such film is specifically, for example, an acrylic polymer, polyethylene, an ethylene-vinyl acetate copolymer, polyurethane, polyether polyester,or a polyamide derivative. Of those, the acrylic polymer, polyurethane, polyether polyester, and the polyamide derivative are preferred. This is because of the following reasons. That is, a film made of any such material is excellent in water vapor permeability, and hence the respiration of the skin covered with the film is inhibited to a small extent and the albinism of the skin can be suppressed. Further, the filmhas excellent transparency, and hence one can attach the tape while viewing an attachment place (for example, upon fixation of a punctured catheter, while observing a catheter-inserted portion).

Any appropriate thickness can be adopted as the thickness of the above-mentioned film base material depending on purposes and applications. The thickness of the film base material is preferably 1 µm to 150 µm, more preferably 5 µm to 75 µm, or particularly preferably 10 µm to 50 µm. With such thickness, the film base material is excellent in tracking performance for unevenness on the surface of the living organism, and can favorably function as a base material for the medical pressure-sensitive adhesive tape. In addition, the medical pressure-sensitive adhesive tape of the present invention can be attached to the surface of the living organism with good operability and without the generation of wrinkles even when the film base material is extremely thin as described above.

### B-2. Pressure-sensitive adhesive layers

Any appropriate pressure-sensitive adhesive can be adopted as a pressure-sensitive adhesive of which the above-mentioned first pressure-sensitive adhesive layer is constituted as long as the tape can be used by being attached to the surface of a living organism. Specific examples of such pressure-sensitive adhesive include natural rubber-based, synthetic rubber-based, acrylic, silicone-based, and hot melt-based pressure-sensitive adhesives. Of those, the acrylic pressure-sensitive adhesives are preferred. This is because the transparency of the medical pressure-sensitive adhesive tape can be additionally improved and a stimulus applied to a skin can be additionally suppressed. Of the acrylic pressure-sensitive adhesives, an oily gel, acrylic pressure-sensitive adhesive is particularly preferred. This is because low stimuli are applied even to a scarred portion, a seamed wound, and skins suffering fromphotosensitivity and a photo allergy. Details about the oily gel, acrylic pressure-sensitive adhesive are described in, for example, Japanese Patent Application Laid-open No. Hei 06-319793, and its description is incorporated herein by reference.

The above-mentioned first pressure-sensitive adhesive layer may contain any appropriate transdermally absorbable drug as required. The medical pressure-sensitive adhesive tape in which the transdermally absorbable drug is incorporated into the above-mentioned first pressure-sensitive adhesive layer can be suitably used as a transdermal absorption tape formulation. Examples of the transdermally absorbable drug include drugs that can be transdermally absorbed such as corticosteroids, analgesic anti-inflammatory drugs, hypnotic depressants, tranquilizers, antihypertensive drugs, hypotensive diuretic drugs, antibiotics, anesthetic drugs, antibacterial drugs, antifungal drugs, vitamin drugs, coronary vasodilators, antihistaminic drugs, antitussive drugs, sex hormones, antidepressants, cerebral circulation activators, antiemetic drugs, antitumor drugs, and biological drugs. One kind of those transdermally absorbable drugs may be used alone, or two or more kinds of them may be used in combination.

The content of the above-mentioned transdermally absorbable drug can be set to any appropriate content as required (depending on, for example, the kind of the transdermally absorbable drug and a purpose of administration). The content of the above-mentioned transdermally absorbable drug is preferably 1 wt% to 40wt%, or more preferably 3 wt% to 30wt% in the above-mentioned pressure-sensitive adhesive layer. When the content falls within such range, the medical pressure-sensitive adhesive tape of the present invention can discharge the drug in an amount effective and sufficient for therapy.

Any appropriate thickness can be adopted as the thickness of the above-mentioned first pressure-sensitive adhesive layer depending on purposes and applications. The thickness of the above-mentioned first pressure-sensitive adhesive layer is preferably 5 µm to 400 µm, more preferably 10 µm to 300 µm, or particularly preferably 30 µm to 200 µm when the first pressure-sensitive adhesive layer contains the above-mentioned transdermally absorbable drug. The thickness of the above-mentioned first pressure-sensitive adhesive layer is preferably 20 µm to 100 µm, or more preferably 30 µm to 70 µm when the first pressure-sensitive adhesive layer does not contain the above-mentioned transdermally absorbable drug. When the thickness of the above-mentioned pressure-sensitive adhesive layer falls within such range, the floating and release of the attached medical pressure-sensitive adhesive tape can be favorably prevented because the tape is excellent in adhesiveness with the surface of a living organism. In addition, the curling of an end of the medical pressure-sensitive adhesive tape when the medical pressure-sensitive adhesive tape is attached for a long time period can also be favorably prevented.

The same pressure-sensitive adhesive as the pressure-sensitive adhesive of which the above-mentioned first pressure-sensitive adhesive layer is constituted can be used as a pressure-sensitive adhesive of which each of the second pressure-sensitive adhesive layer and the third pressure-sensitive adhesive layer described above is constituted.

Any appropriate thickness can be adopted as the thickness of the above-mentioned second pressure-sensitive adhesive layer depending on purposes and applications. The thickness of the above-mentioned second pressure-sensitive adhesive layer is preferably 5 µm to 100 µm, more preferably 10 µm to 70 µm, or particularly preferably 30 µm to 70 µm.

Any appropriate thickness can be adopted as the thickness of the above-mentioned third pressure-sensitive adhesive layer depending on purposes and applications. The thickness of the above-mentioned third pressure-sensitive adhesive layer is preferably 5 µm to 60 µm, or more preferably 10 µm to 50 µm. When the thickness of the above-mentioned third pressure-sensitive adhesive layer falls within such range, the medical pressure-sensitive adhesive tape is excellent in adhesiveness with the surface of a living organism. Accordingly, when the third pressure-sensitive adhesive layer contacts the living organism after the main body of the medical pressure-sensitive adhesive tape has been attached, the floating and release of the attached medical pressure-sensitive adhesive tape can be favorably prevented. In addition, the curling of an end of the medical pressure-sensitive adhesive tape when the medical pressure-sensitive adhesive tape is attached for a long time period can also be favorably prevented.

Any appropriate method can be adopted as a method of providing the first pressure-sensitive adhesive layer, the second pressure-sensitive adhesive layer, and the third pressure-sensitive adhesive layer described above. Specific examples of the method include a method involving applying a pressure-sensitive adhesive composition to a surface on which a pressure-sensitive adhesive layer is placed and drying the applied composition, and a method involving transferring and laminating the pressure-sensitive adhesive layer molded out of the pressure-sensitive adhesive composition into a predetermined shape onto the surface on which the pressure-sensitive adhesive layer is placed.

### B-3. Release liner

Any appropriate release liner can be adopted as the above-mentioned release liner as long as the release liner can be favorably released from any pressure-sensitive adhesive layer used in the present invention. The release liner representatively has a base body and a release treatment layer formed on the surface of the base body. Specific examples of the base body include a plastic film (such as polyethylene, polypropylene, polyester, or a laminated composite of them) and paper (such as woodfree paper or kraft paper) . The release treatment layer can be formed by subjecting the surface of the base body to, for example, a silicone-based resin treatment or a fluorine-based resin treatment. In the present invention, the release treatment layer is preferably formed on each of both surfaces of the base body.

The thickness of the above-mentioned release liner can be appropriately set depending on purposes. The thickness of the release liner is preferably 50 µm to 250 µm, or more preferably 75 µm to 200 µm.

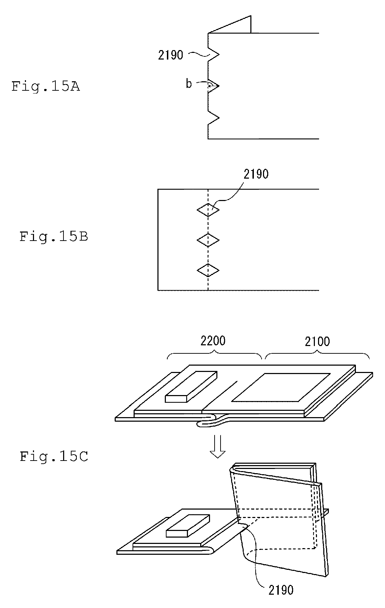

When the medical pressure-sensitive adhesive tape of the example has a cut portion, the above-mentioned release liner preferably has notches **2190** on the above-mentioned folding lines as illustrated in each of FIGS. **15A** and **15B.** FIG. **15A** is a view illustrating the notches **2190** in a state in which the release liner is folded back, and FIG. **15B** is a view illustrating the notches **2190** in a state in which the release liner is unfolded. When the above-mentioned release liner has notches, in the case where the above-mentioned release liner is divided into two pieces, and a first divided piece and a second divided piece overlap each other (that is, in the case of the examples illustrated in FIG. **11**), upon opening of the cut portion, one divided piece retains the other divided piece with the notches as illustrated in FIG. **15C**. As a result, a state in which the cut portion is opened can be stably maintained. Figures 15A to 15C show examples useful for understanding the invention.

Any appropriate shape can be adopted as the shape of each of the above-mentioned notches as long as such effect is obtained. Specific examples of the shape of each of the above-mentioned notches include a triangle, a linear shape, and a rectangular shape (the triangle in the illustrated example). A depth **b** of each of the above-mentioned notches is preferably 2 mm to 6 mm, or more preferably 3 mm to 5 mm. The depth **b** of each of the above-mentioned notches more preferably coincides with the overlapping width between the above-mentioned first divided piece and the above-mentioned second divided piece. When the depth **b** of each of the notches falls within such range, the state in which the cut portion is opened can be stably maintained, and the first divided piece and the second divided piece described above can be easily separated from each other upon closing of the cut portion. The above-mentioned notches may be provided only for the first divided piece, may be provided only for the second divided piece, or may be provided for both the first divided piece and the second divided piece.

### B-4. Support

Any appropriate material can be used in the above-mentioned support as long as the effect of the present invention is obtained. Specific examples of the material for the support include cotton, rayon, nylon, urethane, polyethylene, polypropylene, polyester, and pulp. As any such material has a moderate strength and flexibility, a medical pressure-sensitive adhesive tape having good shape retentivity and excellent in tracking performance for unevenness on the surface of a living organism can be obtained. An additionally specific form of the above-mentioned support is, for example, a spun-laced nonwoven fabric, a spunlaid nonwoven fabric, a meltblown nonwoven fabric, a urethane nonwoven fabric, a urethane foam sheet, a polyethylene foam sheet, a polypropylene foam sheet, or a mixed nonwoven fabric of pulp and an acrylic ester. The above-mentioned support may be of a single-layer structure, or may be of a laminated structure formed of two or more layers. In the case of the laminated structure, the respective layers may be identical to or different from each other.

The thickness of the above-mentioned support can be set to any appropriate thickness depending on purposes. The thickness of the above-mentioned support is preferably 100 µm to 500 µm, or more preferably 200 µm to 400 µm. When the thickness of the above-mentioned support falls within such range, a medical pressure-sensitive adhesive tape excellent in balance between its strength and flexibility, having good shape retentivity, and excellent in tracking performance for unevenness on the surface of a living organism can be obtained.

The above-mentioned support preferably has a window portion as illustrated in, for example, each of FIGS. **5** and **10**. When the above-mentioned support has the window portion, in the case where the medical pressure-sensitive adhesive tape covers an affected area in the surface of a living organism, the medical pressure-sensitive adhesive tape can be attached while the affected area is observed. In addition, in the case where the medical pressure-sensitive adhesive tape is used as a film dressing for fixing a medical instrument such as a catheter, the film dressing can be attached while a puncture portion is observed. Any appropriate shape and size can be adopted as the shape and size of the above-mentioned window portion as long as the affected area or the position at which the medical instrument is fixed can be observed.

### B-5. Fixing material

The medical pressure-sensitive adhesive tape preferably has a fixing material in certain examples. The medical pressure-sensitive adhesive tape having the fixing material as described above can be suitably used as a film dressing capable of fixing a medical instrument. Specific examples of the medical pressure-sensitive adhesive tape having the fixing material (film dressing) include the medical pressure-sensitive adhesive tapes illustrated in FIGS. **10** to **14**. The presence of the fixing material can provide a medical pressure-sensitive adhesive tape excellent in operability and capable of favorably fixing a medical instrument such as a catheter at its puncture portion. To be specific, providing the fixing material enables simultaneous location of the medical instrument such as a catheter and the medical pressure-sensitive adhesive tape. In addition, the fixing material aids the fixation of the medical instrument such as a catheter, and hence excellent fixing performance is obtained and a difference in fixing performance resulting from operator shardly occurs. Further, the amount in which the tape is used for aiding fixation can be reduced because of such excellent fixing performance. As a result, a cost reduction and a reduction in burden on a skin to which the medical pressure-sensitive adhesive tape is applied can be achieved.

Any appropriate fixing material can be used as the above-mentioned fixing material as long as the effect of the present example is obtained. Specific examples of the material for the fixing material include cotton, rayon, nylon, urethane, polyethylene, polypropylene, polyester, and acrylic ester, pulp, and rubber. As any such material has a moderate strength and flexibility, a medical pressure-sensitive adhesive tape that suppresses a feeling of being compressed by a medical instrument such as a catheter can be obtained. An additionally specific form of the above-mentioned fixing material is, for example, a spun-laced nonwoven fabric, a spunlaid nonwoven fabric, a meltblown nonwoven fabric, a urethane nonwoven fabric, a urethane foam, a polyethylene foam, a polypropylene foam, or a mixed nonwoven fabric of pulp and an acrylic ester. Of those, the urethane foam and the polyethylene foam are preferred. When the above-mentioned fixing material is formed of the urethane foam or the polyethylene foam, a medical pressure-sensitive adhesive tape excellent in fixing performance for a medical instrument such as a catheter and capable of lowering a physical stimulus of the medical instrument such as a catheter to the surface of a living organism can be obtained. The above-mentioned fixing material may be of a single-layer structure, or may be of a laminated structure formed of two or more layers. In the case of the laminated structure, the respective layers may be identical to or different from each other.

The above-mentioned fixing material is preferably provided with a insection. When the insection is provided, the medical instrument such as a catheter can be fixed in an additionally favorable fashion. The above-mentioned insection is provided at any appropriate position so as to have any appropriate shape as long as the medical instrument such as a catheter can be favorably fixed. The position at which the above-mentioned insection is provided is, for example, the surface of the fixing material opposite to the support, the side surface of the fixing material on the side of a boundary between the above-mentioned film portion and the above-mentioned fixation aid portion, or a corner formed from these surfaces. The sectional shape of the above-mentioned insection is, for example, a triangle, a quadrangle, or a semicircle.

The thickness of the above-mentioned fixing material is preferably 1 mm to 13 mm, or more preferably 2 mm to 8 mm. When the thickness of the above-mentioned fixing material is smaller than 1 mm, unevenness on a bonding portion between the puncture portion and transfusion tube of the medical instrument cannot be absorbed, and hence sufficient fixing performance may not be obtained. In addition, the strength of the fixing material may be insufficient. When the thickness of the above-mentioned fixing material is larger than 13 mm, the bonding portion between the puncture portion and the transfusion tube cannot be stably placed on the upper surface of the fixing material, and hence sufficient fixing performance may not be obtained. In addition, the fixing material is so thick that the portion where the medical instrument is fixed is apt to hitch on a cloth, a bed, or the like, and a needle may be extracted.

Each side in the widthwise direction of the above-mentioned fixing material (that is, the direction perpendicular to the boundary between the above-mentioned film portion and the above-mentioned fixation aid portion) has a length of preferably 5 mm or more, more preferably 5 mm to 50 mm, or particularly preferably 10 mm to 30 mm. When the length of each side in the widthwise direction of the above-mentioned fixing material falls within such range, a medical pressure-sensitive adhesive tape of an appropriate size having a strength enough to fix a medical instrument such as a catheter favorably can be obtained. Each side in the lengthwise direction of the above-mentioned fixing material (that is, the direction parallel to the boundary between the above-mentioned film portion and the above-mentioned fixation aid portion) has a length of preferably 2 mm or more, more preferably 2 mm to 50 mm, or particularly preferably 5 mm to 50 mm. When the length of each side in the lengthwise direction of the above-mentioned fixing material falls within such range, such a medical pressure-sensitive adhesive tape of an appropriate size that a medical instrument such as a catheter hardly falls down from the fixing material can be obtained.

### B-6. Fixing tape

When the medical pressure-sensitive adhesive tape is used for fixing a medical instrument in certain examples (such as the medical pressure-sensitive adhesive tape illustrated in any one of FIGS. **10** to **14**), the above-mentioned fixing tape is attached from above the main body of the medical pressure-sensitive adhesive tape attached to the surface of a living organism so as to aid the fixation of the medical instrument with the medical pressure-sensitive adhesive tape. When the above-mentioned fixing tape is provided integrally with the medical pressure-sensitive adhesive tape, operability upon attachment and fixation of the medical pressure-sensitive adhesive tape can be markedly improved. The fixing tape can have a base body and a pressure-sensitive adhesive layer. Detailed description of the base body is omitted because the constitution of a medical tape or bandage tape typically used in the industry is adopted for the base body. The pressure-sensitive adhesive layer is as described in the above section B-2.

### C. Specific procedure for attaching medical pressure-sensitive adhesive tape

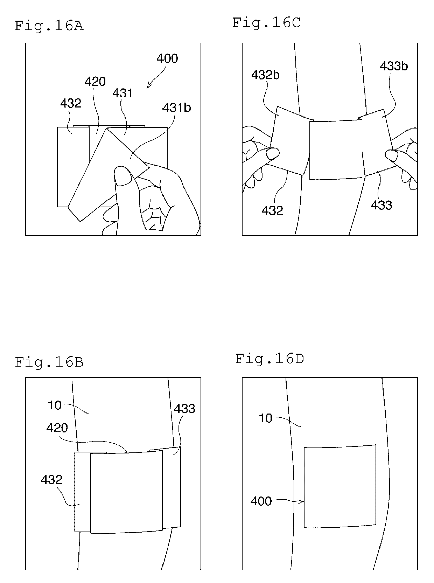

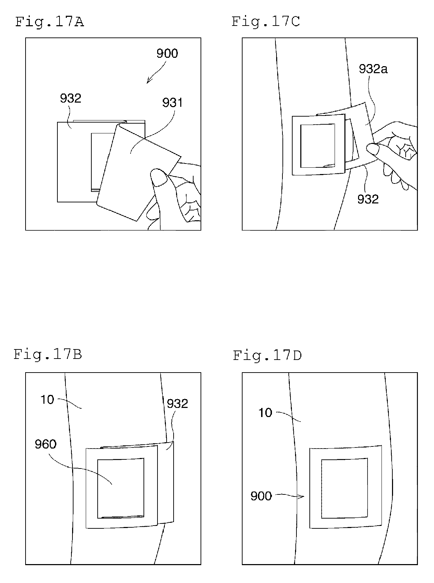

FIGS. **16** are schematic diagrams for illustrating a specific attachment procedure for the medical pressure-sensitive adhesive tape **400** of the embodiment illustrated in FIG. **4**. FIGS. **17** are schematic diagrams for illustrating a specific attachment procedure for the medical pressure-sensitive adhesive tape **900** of the embodiment illustrated in FIG. **9**. An attachment procedure for the medical pressure-sensitive adhesive tape of the present invention is specifically described by taking those embodiments as representative examples.

In the attachment of the medical pressure-sensitive adhesive tape **400** of the present invention, as illustrated in FIG. **16A****,** the first divided piece **431** of the release liner is released from the main body of the medical pressure-sensitive adhesive tape first. In this case, when the first divided piece **431** has an extending portion, the first divided piece **431** is released in the widthwise direction of the medical pressure-sensitive adhesive tape **400** (that is, the right direction of the space of each of FIGS. **16**) with the extending portion as a holding margin (with the extending portion **431b** as a holding margin in the illustrated example). Next, as illustrated in FIG. **16B****,** part of the first pressure-sensitive adhesive layer **420** exposed as a result of the release of the first divided piece 431 is attached to a living organism surface **10**. After that, as illustrated in FIG. **6C**, the second divided piece **432** and third divided piece **433** of the release liner are released from the main body of the medical pressure-sensitive adhesive tape. In this case, the second divided piece **432** and third divided piece **433** of the release liner are released in the widthwise directions of the medical pressure-sensitive adhesive tape **400** (that is, the left and right directions of the space of each of FIGS. **16**) with the folding portions **432a** and **433a** (or when the folding portions are provided with extending portions as illustrated in FIG. **4**, the extending portions **432b** and **433b**) as holding margins. As illustrated in FIG. **16D**, the pressure-sensitive adhesive layer **420** exposed as a result of the release of the second divided piece **432** and the third divided piece **433** as described above is attached to the living organism surface **10**. Thus, the attachment of the main body of the medical pressure-sensitive adhesive tape (a laminate of the film base material **410** and the first pressure-sensitive adhesive layer **420**) is completed. The medical pressure-sensitive adhesive tape of the present invention is excellent in operability and can be attached to the living organism surface without the generation of wrinkles because the attachment of the main body of the medical pressure-sensitive adhesive tape can be performed in a stepwise manner as described above.

In the attachment of the medical pressure-sensitive adhesive tape **900** of the present invention, as illustrated in FIG. **17A**, the first divided piece **931** of the release liner is released from the main body of the medical pressure-sensitive adhesive tape first. In this case, when the first divided piece **931** has an extending portion, the first divided piece **931** is released in the widthwise direction of the medical pressure-sensitive adhesive tape **900** (that is, the right direction of the space of each of FIGS. **17**) with the extending portion as a holding margin. Next, as illustrated in FIG. **17B**, part of the first pressure-sensitive adhesive layer exposed as a result of the release of the first divided piece **931** (that is, the portion of the first pressure-sensitive adhesive layer including the portion corresponding to the window portion **960** and not covered with the second divided piece **932**) is attached to the living organism surface **10**. After that, as illustrated in FIG. **17C****,** the second divided piece **932** of the release liner is released from the main body of the medical pressure-sensitive adhesive tape. In this case, the second divided piece **932** of the release liner is released in the widthwise direction of the medical pressure-sensitive adhesive tape **900** (that is, the right direction of the space of each of FIGS. **17**) with the folding portion **932a** (or when the folding portion is provided with an extending portion, the extending portion) as a holding margin. As illustrated in FIG. **17D**, the pressure-sensitive adhesive layer exposed as a result of the release of the second divided piece **932** as described above (that is, the pressure-sensitive adhesive layer corresponding to the frame portion of the support **940** covered with the second divided piece **932**) is attached to the living organism surface **10**. Thus, the attachment of the main body of the medical pressure-sensitive adhesive tape is completed. Although not illustrated, the following procedure may be adopted. That is, in the above-mentioned operation of FIG. **17A**, part of the first divided piece **931** is released from the main body of the medical pressure-sensitive adhesive tape, and in the above-mentioned operation of FIG. **17B**, the first pressure-sensitive adhesive layer thus exposed is attached to the living organism surface **10**. After that, in the above-mentioned operation of FIG. **17C**, the entirety of the first divided piece **931** and the second divided piece **932** are released.

### (Specific procedure for attaching film dressing)

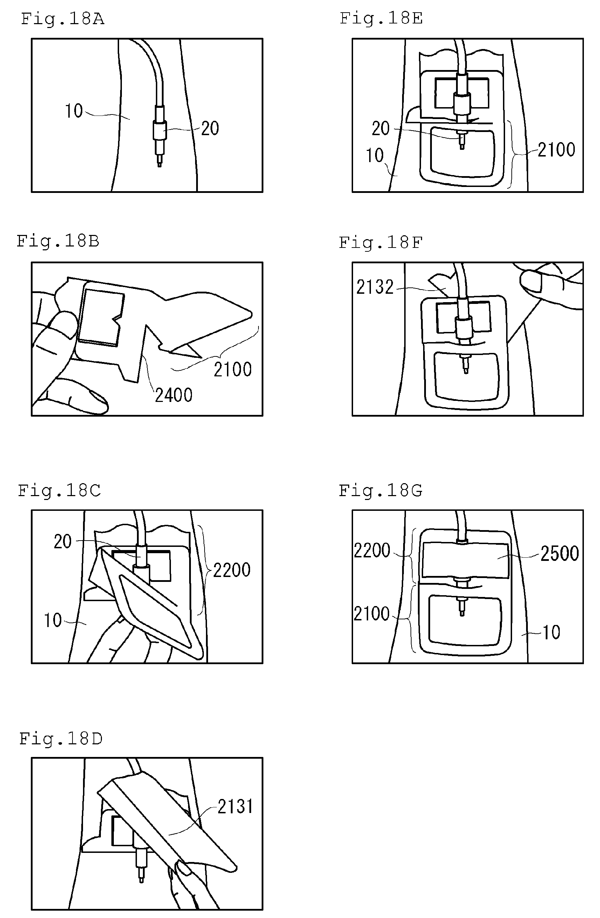

FIGS. **18** are schematic diagrams for illustrating a specific attachment procedure for a medical pressure-sensitive adhesive tape (representatively a film dressing) in the case where a cut portion is provided and a release liner is divided into two pieces, according to an example useful for understanding the invention. First, as illustrated in FIG. **18A****,** a medical instrument (catheter in the illustrated example) **20** is punctured in the living organism surface **10,** and the medical pressure-sensitive adhesive tape (filmdressing) is attached in the state. Hereinafter, specific description is given by taking the medical pressure-sensitive adhesive tape (film dressing) illustrated in FIG. **11** as a representative example.

When the release liner is divided into two pieces, in the attachment of the above-mentioned medical pressure-sensitive adhesive tape **2000,** an end of a film portion **2100** is twisted first so that the film portion **2100** and a fixation aid portion **2200** may each be brought into a distorted state. As a result, as illustrated in FIG. **18B****,** a cut portion **2400** is brought into an open state. In this case, when the release liner has a notch **2190** as described in the above section B-3, the state in which the cut portion **2400** is opened can be stably maintained. Next, as illustrated in FIG. **18C****,** the fixation aid portion **2200** is placed between the medical instrument **20** and the living organism surface **10,** and hence the film portion **2100** is brought into a state of being positioned above the medical instrument **20.** That is, the medical instrument **20** is fixed on the fixation aid portion **2200,** and is brought into a state of being punctured in the living organism surface **10** below the film portion **2100** while squeezing through the cut portion **2400.**

Next, as illustrated in FIG. **18D****,** the first divided piece **2131** (that is, the release liner in the film portion) is released in the downward direction of the space. Then, as illustrated in FIG. **18E**, the film portion **2100** is attached to the living organism surface **10** so as to cover the puncture portion of the medical instrument **20.** Further, as illustrated in FIG. **18F**, the second divided piece **2132** (that is, the release liner in the fixation aid portion) is released in the upward direction of the space. Then, as illustrated in FIG. **18G****,** the fixation aid portion **2200** is attached to the living organism surface **10.** Further, as required, a fixing tape **2500** is released from the release liner, and is then attached so as to cover the fixing material. Thus, the attachment of the medical pressure-sensitive adhesive tape **2000** is completed.

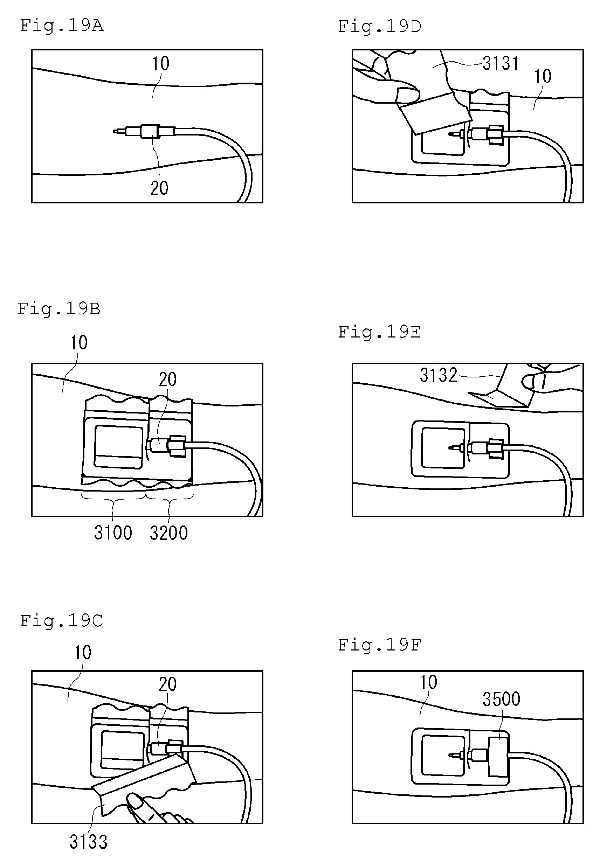

FIGS. **19** are schematic diagrams for illustrating a specific attachment procedure for a medical pressure-sensitive adhesive tape (representatively a film dressing) in the case where a cut portion is provided and a release liner is divided into three pieces, according to an example useful for understanding the invention. First, as illustrated in FIG. **19A****,** the medical instrument (catheter in the illustrated example) **20** is punctured in the living organism surface **10,** and the medical pressure-sensitive adhesive tape (filmdressing) is attached in the state. Hereinafter, specific description is given by taking the medical pressure-sensitive adhesive tape (film dressing) illustrated in FIG. **12** as a representative example.

When the release liner is divided into three pieces, in the attachment of the above-mentioned medical pressure-sensitive adhesive tape **3000,** an end of a film portion **3100** is twisted first so that the film portion **3100** and a fixation aid portion **3200** may each be brought into a distorted state, and a cut portion **3400** may be brought into an open state. Next, as illustrated in FIG. **19B****,** the fixation aid portion **3200** is placed between the medical instrument **20** and the living organism surface **10,** and hence the film portion **3100** is brought into a state of being positioned above the medical instrument **20.** That is, the medical instrument **20** is fixed on the fixation aid portion **3200,** and is brought into a state of being punctured in the living organism surface **10** below the film portion **3100** while squeezing through the cut portion **3400.**

Next, as illustrated in FIG. **19C****,** the fourth divided piece **3134** is released in the downward direction of the space so that the portions of the film portion **3100** and the fixation aid portion **3200** corresponding to the fourth divided piece **3134** may be attached to the living organism surface **10.** Further, as illustrated in FIG. **19D****,** the first divided piece **3131** is released in the upward direction of the space, and the remaining portion of the film portion **3100** is attached to the living organism surface **10** so as to cover the puncture portion of the medical instrument **20.** Next, as illustrated in FIG. **19E****,** the second divided piece **3132** is released in the upward direction of the space. Then, as illustrated in FIG. **19F**, the remaining portion of the fixation aid portion **3200** is attached to the living organism surface **10.** Further, as required, a fixing tape **3500** is released from the release liner, and is then attached so as to cover the fixing material. Thus, the attachment of the medical pressure-sensitive adhesive tape **3000** is completed.

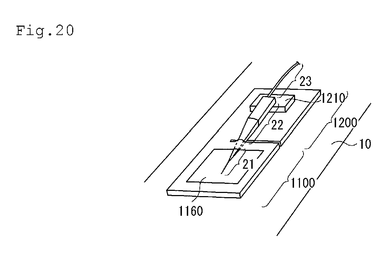

FIG. **20** is a schematic perspective diagram for illustrating a state in which the main body of the medical pressure-sensitive adhesive tape illustrated in each of FIGS. **10** is attached to the surface of a living organism, according to an example useful for understanding the invention. As illustrated in FIG. **20**, at the time of the attachment of the medical pressure-sensitive adhesive tape illustrated in each of FIGS. **10****,** the tape is attached to the living organism surface **10** so that the film portion **1100** may cover a puncture portion **21** of a medical instrument such as a catheter. In addition, the fixation aid portion **1200** is attached to the living organism surface **10.** At least one of a bonding portion **22** between the transfusion tube and puncture portion of the medical instrument such as a catheter, and a transfusion tube **23** is placed on the fixing material **1210,** and the fixation aid portion aids the fixation of the bonding portion and the tube. When a fixing tape is used, the fixing tape is preferably attached so as to cover the fixing material **1210,** the bondingportion **22** between the transfusion tube and puncture portion of the medical instrument such as a catheter, and the transfusion tube **23** in the fixation aid portion **1200,** though the fixing tape is not illustrated. When the medical instrument such as a catheter is fixed in such state, the leakage of a drug solution to the outside of a blood vessel due to the movement of the indwelling needle of a catheter, the risk of the extraction of the indwelling needle, and the floating and release of the medical pressure-sensitive adhesive tape due to the movement of the catheter can be favorably prevented.

### D. Applications

The medical pressure-sensitive adhesive tape of the present invention can be used in, for example, a film dressing, a bandage, or a transdermal absorption tape formulation.

The medical pressure-sensitive adhesive tape of the present invention can be used as a bandage by providing part, or the entirety, of the surface of the above-mentioned first pressure-sensitive adhesive layer on the side of the above-mentioned release liner with a pad for protecting a wounded portion in the surface of a living organism. A material showing liquid-absorbing performance is a preferred material used in the above-mentioned pad. Specific examples of the material used in the above-mentioned pad include a gauze, a liquid absorbable foam, a cotton fabric, a nonwoven fabric, a composite of an absorbent cotton and a nonwoven fabric, and a composite of an absorbent cotton and a knitted net.

The medical pressure-sensitive adhesive tape of the present invention can be used as a transdermal absorption tape formulation by incorporating a transdermally absorbable drug into the above-mentioned first pressure-sensitive adhesive layer. Examples of the transdermally absorbable drug include the drugs described in the section B-2.

### [Example 1] (Production of medical pressure-sensitive adhesive tape 1)

### 1. Lamination of film base material and first pressure-sensitive adhesive layer

A polyurethane film having a thickness of 30 µm was used as a film base material.

A pressure-sensitive adhesive layer (first pressure-sensitive adhesive layer) formed of an acrylic pressure-sensitive adhesive was provided for the surface of the film base material so as to have a thickness of 30 µm. Thus, a laminate 1 of the film base material and the first pressure-sensitive adhesive layer was obtained. The laminate 1 was of a size measuring 80 mm by 80 mm.

### 2. Lamination of support and second pressure-sensitive adhesive layer

A spun-laced nonwoven fabric having a thickness of 300 µm was used as a support. The support was provided with a window portion measuring 60 mm (widthwise direction) by 70 mm (lengthwise direction) with a spring cutter at a position distant from each of both widthwise-direction ends by 10 mm and from each of both lengthwise-direction ends by 5 mm. A pressure-sensitive adhesive layer (second pressure-sensitive adhesive layer) formed of an acrylic pressure-sensitive adhesive was provided for the surface of the support so as to have a thickness of 50 µm. Thus, a laminate 2 of the support and the second pressure-sensitive adhesive layer was obtained. The laminate 2 was of a size measuring 80 mm by 80 mm.

### 3. Attachment of laminate 1, laminate 2, and release liner

One surface of paper having a thickness of 100 µm was subjected to a silicone resin treatment. Thus, a release liner was obtained. The resultant release liner was divided into a first divided piece and a second divided piece.

The second divided piece was of a size measuring 150 mm (widthwise direction) by 80 mm (lengthwise direction). The second divided piece was attached to the above-mentioned laminate 1 so as to cover a width of 70 mm on one widthwise-direction end of the first pressure-sensitive adhesive layer of the laminate 1, and was provided with a folding portion of 80 mm in width in the widthwise direction (in other words, an extending portion was of 10 mm in width). In this case, the second divided piece was provided with a window portion so that the second divided piece did not cover the portion of the first pressure-sensitive adhesive layer corresponding to the window portion.

The first divided piece was of a size measuring 100 mm (widthwise direction) by 80 mm (lengthwise direction). The first divided piece was attached to the laminate 1 so as to cover the portion of the first pressure-sensitive adhesive layer that is not covered with the above-mentioned second divided piece (portion having a width of 10 mm at the other widthwise-direction end of the first pressure-sensitive adhesive layer), and further, was provided with an extending portion of 90 mm in width in the widthwise direction. The extending portion was placed so as to cover the second divided piece and the portion of the first pressure-sensitive adhesive layer corresponding to the window portion. In this case, the extending portion of the first divided piece extended toward the outside of the extending portion of the second divided piece by 10 mm. It should be noted that a perforation was provided for the first divided piece at a position distant from the other widthwise-direction end of the first pressure-sensitive adhesive layer by 25 mm so as to be substantially parallel to a side at the widthwise-direction end.

Next, the film base material of the laminate 1 to which the release liner had been attached as described above and the second pressure-sensitive adhesive layer of the above-mentioned laminate 2 were attached to each other.

Thus, a medical pressure-sensitive adhesive tape 1 having the support, the second pressure-sensitive adhesive layer, the film base material, the first pressure-sensitive adhesive layer, and the release liner (the first divided piece and the second divided piece) in the stated order was obtained.

### [Example 2] (Production of medical pressure-sensitive adhesive tape 2)

### 1. Lamination of film base material and first pressure-sensitive adhesive layer

A polyurethane film having a thickness of 30 µm was used as a film base material.

A pressure-sensitive adhesive layer (first pressure-sensitive adhesive layer) formed of an acrylic pressure-sensitive adhesive was provided for the surface of the film base material so as to have a thickness of 30 µm. Thus, a laminate 3 of the film base material and the first pressure-sensitive adhesive layer was obtained. The laminate 3 was of a size measuring 80 mm by 80 mm.

### 2. Lamination of laminate 3 and release liner

One surface of paper having a thickness of 100 µm was subjected to a silicone resin treatment. Thus, a release liner was obtained. The resultant release liner was divided into a first divided piece, a second divided piece, and a third divided piece.

The second divided piece and the third divided piece were each of a size measuring 50 mm (widthwise direction) by 80 mm (lengthwise direction). The second divided piece was attached to the above-mentioned laminate 3 so as to cover a width of 25 mm on one widthwise-direction end of the pressure-sensitive adhesive layer of the laminate 3, and was provided with a folding portion of 25 mm in width in the widthwise direction (in other words, no extending portion was provided). The third divided piece was attached to the above-mentioned laminate 3 so as to cover a width of 25 mm on the other widthwise-direction end of the pressure-sensitive adhesive layer of the laminate 3, and was provided with a folding portion of 25 mm in width in the widthwise direction (in other words, no extending portion was provided).

The first dividedpiece was of a size measuring 30 mm (widthwise direction) by 80 mm (lengthwise direction). The first divided piece was attached to the laminate 3 so as to cover the portion of the pressure-sensitive adhesive layer that is not covered with the second divided piece and the third divided piece described above.

Thus, a medical pressure-sensitive adhesive tape 2 including the film base material, the pressure-sensitive adhesive layer, and the release liner (the first divided piece, the second divided piece, and the third divided piece) in the stated order was obtained.

### [Example 3] (Production of medical pressure-sensitive adhesive tape 3)

A medical pressure-sensitive adhesive tape 3 was obtained in the same manner as in Example 2 except that the second divided piece and the third divided piece were each of a size measuring 55 mm (widthwise direction) by 80 mm (lengthwise direction), and the folding portion of each of the second divided piece and the third divided piece was of 30 mm in width (in other words, an extending portion was of 5 mm in width).

### [Example 4] (Production of medical pressure-sensitive adhesive tape 4)

A medical pressure-sensitive adhesive tape 4 was obtained in the same manner as in Example 1 except that the second divided piece and the third divided piece were each of a size measuring 55 mm (widthwise direction) by 80 mm (lengthwise direction), the folding portion of each of the second divided piece and the third divided piece was of 30 mm in width (in other words, an extending portion was of 5 mm in width) , the first divided piece was of a size measuring 90 mm (widthwise direction) by 80 mm (lengthwise direction), and an extending portion of 30 mm in width was provided for each of both widthwise-direction sides of the first divided piece. In this case, the extending portions of the first divided piece did not extend toward the outsides of the extending portions of the second divided piece and the third divided piece.

### [Example 5] (Production of medical pressure-sensitive adhesive tape 5)

A medical pressure-sensitive adhesive tape 5 was obtained in the same manner as in Example 1 except that the second divided piece and the third divided piece were each of a size measuring 55 mm (widthwise direction) by 80 mm (lengthwise direction), the folding portion of each of the second divided piece and the third divided piece was of 30 mm in width (in other words, an extending portion was of 5 mm in width) , the first divided piece was of a size measuring 100 mm (widthwise direction) by 80 mm (lengthwise direction), and an extending portion of 35 mm in width was provided for each of both widthwise-direction sides of the first divided piece. In this case, the extending portions of the first divided piece extended 5 mm toward the outsides of the extending portions of the second divided piece and the third divided piece.

### [Example 6] (Production of film dressing 1)

### 1. Laminate 4 of film base material and first pressure-sensitive adhesive layer

A polyurethane film having a thickness of 30 µm was used as a film base material.

A pressure-sensitive adhesive layer (first pressure-sensitive adhesive layer) formed of an acrylic pressure-sensitive adhesive was provided for the surface of the film base material so as to have a thickness of 30 µm. Thus, a laminate 4 of the film base material and the first pressure-sensitive adhesive layer was obtained. The laminate 4 was of a size measuring 40 mm (widthwise direction) by 50 mm (lengthwise direction).

### 2. Laminate 5 of support and third pressure-sensitive adhesive layer

A spun-laced nonwoven fabric having a thickness of 300 µm was used as a support.

A pressure-sensitive adhesive layer (third pressure-sensitive adhesive layer) formed of an acrylic pressure-sensitive adhesive was provided for the surface of the support so as to have a thickness of 50 µm. Thus, a laminate 5 of the support and the third pressure-sensitive adhesive layer was obtained. The laminate 5 was of a size measuring 130 mm (widthwise direction) by 50 mm (lengthwise direction). Further, the laminate 5 was provided with a window portion measuring 30 mm (widthwise direction) by 40 mm (lengthwise direction) with a spring cutter at a position distant from one widthwise-direction end by 5 mm and from each of both lengthwise-direction ends by 5 mm.

### 3. Laminate 10 of support, third pressure-sensitive adhesive layer, film base material, and first pressure-sensitive adhesive layer

One widthwise-direction end of the laminate 4 and one widthwise-direction end of the laminate 5 were aligned with each other, and the third pressure-sensitive adhesive layer of the above-mentioned laminate 5 and the film base material of the above-mentioned laminate 4 were attached to each other so that the film base material was laminated at the position corresponding to the above-mentioned window portion. Thus, a laminate 10 (measuring 130 mm (widthwise direction) by 50 mm (lengthwise direction)) was obtained. The portion of the laminate 10 corresponding to a film portion was of a size measuring 40 mm (widthwise direction) by 50 mm (lengthwise direction), and the portion of the laminate corresponding to a fixation aid portion was of a size measuring 90 mm (widthwise direction) by 50 mm (lengthwise direction).

Further, a cut portion having a length of 30 mm was provided at the position corresponding to the inner end of the above-mentioned film base material so as to be perpendicular from one lengthwise-direction end of the laminate 10.

### 4. Release liner

One surface of paper having a thickness of 100 µm was subjected to a silicone resin treatment. Thus, a release liner was obtained. The resultant release liner was divided into a first divided piece and a second divided piece. The first divided piece was of a size measuring 90 mm (widthwise direction) by 50 mm (lengthwise direction), and the second divided piece was of a size measuring 198 mm (widthwise direction) by 50 mm (lengthwise direction).

The widthwise-direction end on the side of the laminate 10 not provided with the film base material (in other words, the portion corresponding to the fixation aid portion) and one widthwise-direction end of the second divided piece were aligned with each other. Then, the second divided piece was folded back at a position distant from the aligned ends by 94 mm so that the third pressure-sensitive adhesive layer and the surface of the second dividedpiece subjected to the silicone resin treatment were attached to each other. In this case, the folding portion of the first divided piece had a width of 104 mm. It should be noted that notches each having a depth of 4 mm and a width of 4 mm illustrated in each of FIGS. 15 were continuously provided for the first divided piece at an interval of 10 mm.

Further, the widthwise-direction end on the side of the laminate 10 provided with the film base material (in other words, the portion corresponding to the film portion) and one widthwise-direction end of the first divided piece were aligned with each other. Then, the first divided piece was folded back at a position distant from the aligned ends by 40 mm so that the first pressure-sensitive adhesive layer and the surface of the first dividedpiece subjected to the silicone resin treatment were attached to each other. In this case, the folding portion of the second divided piece had a width of 50 mm.

The first divided piece and the second divided piece each had an extending portion extending from a widthwise-direction end of the laminate 10 by 10 mm. In addition, the first divided piece and the second divided piece overlapped each other by a width of 4 mm.

### 5. Fixing material

Apolyethylene foam (manufacturedby Sekisui Chemical Company, Limited, Volara ES series, thickness: 5 mm) cut into a size measuring 35 mm in a longitudinal direction by 25 mm in a transverse direction was used as a fixing material. The fixing material was attached to such a position that the shortest length from the cut portion to the end of the fixing material on the side of the cut portion was 45 mm on the support on the side of the laminate 10 not provided with the filmbasematerial (inotherwords, the portion corresponding to the fixationaidportion) through an olef in-based hot melt adhesive so that each longitudinal side of the fixing material was substantially parallel to the widthwise direction of the laminate 10 and each transverse side of the fixing material was substantially parallel to the lengthwise direction of the laminate 10.

### 6. Fixing tape

A pressure-sensitive adhesive layer formed of an acrylic pressure-sensitive adhesive was provided for a spun-laced nonwoven fabric having a thickness of 300 µm so as to have a thickness of 50 µm. Thus, a fixing tape was obtained. The fixing tape was cut into a size measuring 25 mm wide by 50 mm long, and then the outside of the first divided piece folded back as described above (side subjected to the silicone resin treatment) and the pressure-sensitive adhesive layer of the fixing tape were attached to each other.

Thus, a film dressing 1 similar to that illustrated in FIG. 13 was produced except for the presence of a fixing tape portion.

### [Example 7] (Production of film dressing 2)

A film dressing 2 was produced in the same manner as in Example 6 except that the length of the cut portion was set to 40 mm, a polyethylene foam (manufactured by Sekisui Chemical Company, Limited, Volara IF series, thickness: 2 mm) cut into a size measuring 5 mm in a longitudinal direction by 2 mm in a transverse direction was used as a fixing material, and the shortest length from the cut portion to the end of the fixing material on the side of the cut portion was set to 20 mm.

### [Example 8] (Production of film dressing 3)

A film dressing 3 was produced in the same manner as in Example 6 except that the length of the cut portion was set to 35 mm, an acrylic/olef in nonwoven fabric (manufactured by Japan Vilene Company, Ltd., thickness: 2.4 mm) cut into a size measuring 10 mm in a longitudinal direction by 25 mm in a transverse direction was used as a fixing material, and the shortest length from the cut portion to the end of the fixing material on the side of the cut portion was set to 15 mm.

### [Example 9] (Production of film dressing 4)

A film dressing 4 was produced in the same manner as in Example 6 except that the length of the cut portion was set to 25 mm, a polyethylene foam (manufactured by Sekisui Chemical Company, Limited, Volara IF series, thickness: 1 mm) cut into a size measuring 35 mm in a longitudinal direction by 25 mm in a transverse direction was used as a fixing material, and the shortest length from the cut portion to the end of the fixing material on the side of the cut portion was set to 5 mm.

### [Example 10] (Production of film dressing 5)

A film dressing 5 was produced in the same manner as in Example 6 except that the length of the cut portion was set to 45 mm, a polyethylene foam (manufactured by INOAC CORPORATION, trade name: P.E-LITE, thickness: 13 mm) cut into a size measuring 5 mm in a longitudinal direction by 15 mm in a transverse direction was used as a fixing material, and the shortest length from the cut portion to the end of the fixing material on the side of the cut portion was set to 25 mm.

### [Example 11] (Production of film dressing 6)

A film dressing 6 was produced in the same manner as in Example 6 except that the length of the cut portion was set to 10 mm, a polyethylene foam (manufactured by TAKIRON Co., Ltd., KALSOFT, thickness: 3 mm) cut into a size measuring 50 mm in a longitudinal direction by 50 mm in a transverse direction was used as a fixing material, and the shortest length from the cut portion to the end of the fixing material on the side of the cut portion was set to 25 mm.

### [Example 12] (Production of film dressing 7)

A film dressing 7 was produced in the same manner as in Example 6 except that the length of the cut portion was set to 5 mm, a spun-laced nonwoven fabric cut into a size measuring 20 mm in a longitudinal direction by 25 mm in a transverse direction was used as a fixing material, the second divided piece was set to be of a size measuring 95 mm (widthwise direction) by 50 mm (lengthwise direction) and the width of its folding portion was set to 5 mm, the first divided piece was set to be of a size measuring 50 mm by 50 mm, and was not folded back, the first divided piece was made to overlap the second divided piece by a width of 10 mm, and the shortest length from the cut portion to the end of the fixing material on the side of the cut portion was set to 30 mm.

### [Example 13] (Production of film dressing 8)

A film dressing 8 was produced in the same manner as in Example 12 except that the length of the cut portion was set to 48 mm, a polyethylene foam (manufactured by INOAC CORPORATION, trade name: P.E-LITE, thickness: 16 mm) cut into a size measuring 10 mm in a longitudinal direction by 10 mm in a transverse direction was used as a fixing material, and the shortest length from the cut portion to the end of the fixing material on the side of the cut portion was set to 50 mm.

### [Example 14] (Production of film dressing 9)

A film dressing 9 was produced in the same manner as in Example 12 except that the length of the cut portion was set to 48 mm, a polyethylene foam (manufacturedby Sekisui Chemical Company, Limited, Volara IF series, thickness: 2 mm) cut into a size measuring 3 mm in a longitudinal direction by 1.5 mm in a transverse direction was used as a fixing material, and the shortest length from the cut portion to the end of the fixing material on the side of the cut portion was set to 3 mm.

### [Example 15] (Production of film dressing 10)

A film dressing 10 was produced in the same manner as in Example 6 except that no fixing material was used.

### [Comparative Example 1] (Production of medical pressure-sensitive adhesive tape 6)

### 1. Lamination of film base material and pressure-sensitive adhesive layer

A polyurethane film having a thickness of 30 µm was used as a film base material.

A pressure-sensitive adhesive layer formed of an acrylic pressure-sensitive adhesive was provided for the surface of the film base material so as to have a thickness of 30 µm. Thus, a laminate 6 of the film base material and the pressure-sensitive adhesive layer was obtained.

### 2. Lamination of laminate 6 and release liner

One surface of paper having a thickness of 100 µm was subj ected to a silicone resin treatment. Thus, a release liner was obtained. The release liner was attached so as to cover the entirety of the pressure-sensitive adhesive layer of the above-mentioned laminate 6. Thus, a medical pressure-sensitive adhesive tape 6 measuring 80 mm (widthwise direction) by 80 mm (lengthwise direction) was obtained.

### [Comparative Example 2] (Production of medical pressure-sensitive adhesive tape 7)

A medical pressure-sensitive adhesive tape 7 was obtained in the same manner as in Comparative Example 1 except that the release liner was divided into a first divided piece (measuring 40 mm (widthwise direction) by 80 mm (lengthwise direction) ) and a second divided piece (measuring 40 mm (widthwise direction) by 80 mm (lengthwise direction)).

### [Comparative Example 3] (Production of medical pressure-sensitive adhesive tape 8)

A medical pressure-sensitive adhesive tape 8 was obtained in the same manner as in Comparative Example 1 except that the release liner was divided into a first divided piece (measuring 30 mm (widthwise direction) by 80 mm (lengthwise direction)), a second divided piece (measuring 25 mm (widthwise direction) by 80 mm (lengthwise direction), and a third divided piece (measuring 25 mm (widthwise direction) by 80 mm (lengthwise direction)).

### [Comparative Example 4] (Production of film dressing 11)

A pressure-sensitive adhesive layer formed of an acrylic pressure-sensitive adhesive was provided on a polyurethane film having a thickness of 30 µm so as to have a thickness of 30 µm. A release liner was obtained by subjecting one surface of paper having a thickness of 100 µm to a silicone resin treatment, and then the surface of the release liner subjected to the silicone resin treatment and the pressure-sensitive adhesive layer were attached to each other. Thus, a film dressing 11 measuring 50 mm (widthwise direction) by 40 mm (lengthwise direction) was produced.

### <Methods of evaluating medical pressure-sensitive adhesive tape>

The medical pressure-sensitive adhesive tapes obtained in Examples 1 to 5 and Comparative Examples 1 to 3 described above were each attached to a plastic tube of a cylindrical shape having a diameter of 76 mm and a length of 300 mm in conformity with the procedure illustrated in FIGS. 17 for each of Example 1 and Comparative Example 2, the procedure illustrated in FIGS. 16 for each of Examples 2 to 5 and Comparative Example 3 , or such a procedure that the release liner was released before the main body of the medical pressure-sensitive adhesive tape was attached for Comparative Example 1. Then, operability at the time of the attachment and a state after the attachment were evaluated as described below. Table 1 shows the results of evaluation.

### (1) Operability

Evaluation for operability was performed on the basis of the following criteria (i) at the time of the release of the first divided piece of each of Examples 1 to 5, and Comparative Examples 2 and 3, and at the time of the release of the release liner of Comparative Example 1 (operation (a) in each of FIGS. **16A** and **17A**), (ii) at the time of temporal stacking of the main body of each medical pressure-sensitive adhesive tape after the release of the first divided piece (operation (b) in each of FIGS. **16B** and **17B**), and (iii) at the time of the release of the second divided piece and the third divided piece (operation (c) in each of FIGS. **16C** and **17C**).
⊚ : Good operability
○: Trouble-free operability
Δ: Somewhat hard to operate
x: Difficult to operate

### (2) Wrinkles and floating

The presence or absence of wrinkles and floating in a state after the attachment of each medical pressure-sensitive adhesive tape was visually observed.

**Table 1**

| | (i) At time of release of first divided piece | (ii) At time of temporal stacking | (iii) At time of release of second and third divided pieces | Wrinkles and floating |
|---|---|---|---|---|
| Example 1 | ⊚ | ⊚ | ⊚ | Absent |
| Example 2 | ○ | ⊚ | ○ | Slight |
| Example 3 | ○ | ⊚ | ⊚ | Slight |
| Example 4 | ○ | ⊚ | ⊚ | Absent |
| Example 5 | ⊚ | ⊚ | ⊚ | Absent |
| Comparative Example 1 | Δ | × | - | Remarkable |
| Comparative Example 2 | ○ | Δ | Δ | Remarkable |
| Comparative Example 3 | ○ | ○ | Δ | Remarkable |

### <Methods of evaluating film dressing>

A medical catheter (a SHUR-PLUG transfusion set manufactured by TERUMO CORPORATION or a SurFlow Flash 22G manufactured by TERUMO CORPORATION) was applied onto a skin, and then the catheter was covered with each of the film dressings of Examples 6 to 15 in conformity with the procedure illustrated in FIGS. **18**. Further, a fixing tape was attached from above the fixing material and the catheter so that a fixed state was reinforced. In the case of the film dressing of Comparative Example 4 , the release liner was released before the tip of the catheter was covered with a polyurethane film. Operability upon fixation of the catheter and practical fixing performance at the time of the pulling of the tube of the catheter with a hand were evaluated on the basis of the following evaluation criteria. Table 2 shows the results of evaluation.

### (1) Operability

Evaluation was performed on the basis of the same evaluation criteria as those for the operability of the medical pressure-sensitive adhesive tape described above.

### (2) Practical fixing performance

⊚: A sense of stability is achieved.
○: No deviation is observed.
Δ: Nearly no deviation is observed.
x: Deviation is observed.

Further, a tensile strength upon pulling of a catheter was measured and evaluated according to the following procedure.

### (3) Tensile strength

A forearm portion was provided with the catheter, and a film dressing was attached in the same manner as that described above. After the attachment, aging was performed for 30 minutes, and then a tensile strength (shear peel strength) upon pulling of the catheter in the direction parallel to the direction in which the catheter was fixed at a speed of 100 mm/min by 20 mm was measured with an autograph (AG-IS Autograph manufactured by Shimadzu Corporation) .

**Table 2**

| | Operability | Practical fixing performance | Tensile strength (N) |
|---|---|---|---|
| Example 6 | ○ | ⊚ | 3.1 |
| Example 7 | ○ | ⊚ | 3.7 |
| Example 8 | ⊚ | ⊚ | 3.1 |
| Example 9 | ○ | ○ | 3.6 |
| Example 10 | ○ | ⊚ | 3.2 |
| Example 11 | ○ | ⊚ | 3.4 |
| Example 12 | Δ | Δ | 1.9 |
| Example 13 | Δ | Δ | 1.9 |
| Example 14 | Δ | Δ | 2.4 |
| Example 15 | Δ | Δ | 1.7 |
| Comparative Example 4 | × | × | 1.3 |

As the operation of each of the medical pressure-sensitive adhesive tapes (Examples 1 and 3 to 5) in each of which the second divided piece and the third divided piece had extending portions was attained by holding the extending portions, additionally favorable performance of a series of operations from the initiation of the attachment operation to the temporal stacking (ii) was attained.

As shown in each of Examples 1 to 5 (of which Examples 1 and 5 are relevant to the present invention), the medical pressure-sensitive adhesive tape is such that the release liner is divided into three or two pieces, and the second divided piece and the third divided piece have folding portions. Accordingly, the main body of the medical pressure-sensitive adhesive tape can be attached to the surface of a living organism according to a procedure involving (i) releasing the first divided piece, (ii) attaching the medical pressure-sensitive adhesive tape to the surface of the living organism after the release, and (iii) releasing the second divided piece and the third divided piece after the attachment. As a result, each of the operations (i) to (iii) can be performed with good operability. In addition, the medical pressure-sensitive adhesive tape of the present invention can be attached in a state in which wrinkles and floating are suppressed.

The first divided piece of the medical pressure-sensitive adhesive tape can be released in a particularly favorable fashion in the case where the extending portion of the first divided piece extends toward the outsides of the second divided piece and the third divided piece (each of Examples 1 and 5).

The second divided piece and third divided piece of the medical pressure-sensitive adhesive tape can each be released in a particularly favorable fashion in the case where the extending portions of the second divided piece and the third divided piece each extend toward the outside of the film base material (each of Examples 1 and 3 to 5).

The film dressings of Examples 6 to 15 are each excellent in operability because their release liners are each divided into two pieces, and each divided piece has a folding portion and an extending portion. In addition, the film dressings of Examples 6 to 15 are each excellent in operability and fixing performance because the film dressings are each provided with a cut portion. In addition, as shown in each of Examples 6 to 14, the presence of a fixing material can provide a film dressing additionally excellent in fixing performance. Further, as illustrated in each of Examples 6 to 11, the use of a fixing material having a specific thickness and a specific size can provide a film dressing significantly excellent in fixing performance.

In addition, a reattachment operation for each of the film dressings of Examples 6 to 15 was performed with extreme ease because their fixing tapes were easily released.

The medical pressure-sensitive adhesive tape of the present invention can be particularly suitably utilized as: each of a film dressing and a bandage for protecting, for example, a wounded portion in the surface of a living organism; a film dressing for fixing a medical instrument such as a catheter; or a transdermal absorption tape formulation.

## Claims

1. A medical pressure-sensitive adhesive tape (300, 900), comprising:
a film base material (310);
a first pressure-sensitive adhesive layer (320); and
a release liner (331, 332, 931, 932) releasably laminated to cover the first pressure-sensitive adhesive layer in the stated order,
wherein:
the release liner is divided into a first divided piece (331, 931) and a second divided piece (332, 932);
the first divided piece is placed at a widthwise-direction end on one side of the first pressure-sensitive adhesive layer to cover part of the first pressure-sensitive adhesive layer, the first divided piece having an extending portion (331b, 931b) of the first divided piece extending beyond the second divided piece;
the second divided piece is placed to cover a portion of the first pressure-sensitive adhesive layer that is not covered with the first divided piece; and
the second divided piece has a folding portion (332a) extending toward a widthwise-direction end, and
the folding portion of the second divided piece has an extending portion of the second divided piece (332b) extending over and beyond the film base material.

2. A medical pressure-sensitive adhesive tape (400), comprising:
a film base material (410);
a first pressure-sensitive adhesive layer (420); and
a release liner (431, 432, 433) releasably laminated to cover the first pressure-sensitive adhesive layer in the stated order,
wherein :
the release liner is divided into a first divided piece (431), a second divided piece (432), and a third divided piece (433);
the second divided piece is placed at a widthwise-direction end on one side of the first pressure-sensitive adhesive layer to cover part of the first pressure-sensitive adhesive layer;
the third divided piece is placed at a widthwise-direction end on the other side of the first pressure-sensitive adhesive layer to cover part of the first pressure-sensitive adhesive layer;
the first divided piece is placed between the second divided piece and the third divided piece in a widthwise direction to cover a portion of the first pressure-sensitive adhesive layer that is not covered with the second divided piece and the third divided piece, the first divided piece having an extending portion (431b, 431b') of the first divided piece extending beyond the second divided piece and the third divided piece; and
the second divided piece and the third divided piece have folding portions (432a, 433a) extending toward the widthwise-direction ends, and
the folding portion (433a) of the third divided piece has an extending portion of the third divided piece (433b) extending over and beyond the film base material.

3. A medical pressure-sensitive adhesive tape according to claim 1, further comprising:
a support (940); and
a second pressure-sensitive adhesive layer,
wherein:
the support is placed on a side of the film base material opposite to the first pressure-sensitive adhesive layer; and
the second pressure-sensitive adhesive layer is placed between the support and the film base material.

4. A medical pressure-sensitive adhesive tape according to claim 3, wherein the support has a window portion (960).

5. A medical pressure-sensitive adhesive tape according to claim 1, further comprising:
a support having a window portion; and
a second pressure-sensitive adhesive layer,
wherein:
the support having the window portion is placed between the film base material and the first pressure-sensitive adhesive layer; and
the second pressure-sensitive adhesive layer is placed between the film base material and the support having the window portion.

6. A film dressing, which is produced by using the medical pressure-sensitive adhesive tape according to claim 1.

7. A bandage, which is produced by using the medical pressure-sensitive adhesive tape according to claim 1.

8. A transdermal absorption tape formulation, which is produced by using the medical pressure-sensitive adhesive tape according to claim 1.

## Patentansprüche

1. Medizinisches druckempfindliches Heftpflaster (300, 900), umfassend:
ein Folienträgermaterial (310);
eine erste druckempfindliche Klebeschicht (320); und eine Trennlage (331, 332, 931, 932), die trennbar geschichtet ist, um die erste druckempfindliche Klebeschicht in der genannten Reihenfolge zu bedecken,
wobei:
die Trennlage in ein erstes geteiltes Stück (331, 931) und ein zweites geteiltes Stück (332, 932) geteilt ist;
das erste geteilte Stück an einem Ende in Richtung der Breite auf einer Seite der ersten druckempfindlichen Klebeschicht platziert ist, um einen Teil der ersten druckempfindlichen Klebeschicht zu bedecken, wobei das erste geteilte Stück einen erweiternden Bereich (331b, 931b) des ersten geteilten Stücks hat, der über das zweite geteilte Stück hinaus erweitert ist,
das zweite geteilte Stück so platziert ist, dass es einen Bereich der ersten druckempfindlichen Klebeschicht bedeckt, der nicht mit dem ersten geteilten Stück bedeckt ist; und
das zweite geteilte Stück einen Faltbereich (332a) hat, der zu einem Ende in Richtung der Breite hin erweitert ist, und der Faltbereich des zweiten geteilten Stücks einen erweiternden Bereich des zweiten geteilten Stücks (332b) hat, der über das Folienträgermaterial und darüber hinaus erweitert ist.

2. Medizinisches druckempfindliches Heftpflaster (400), umfassend:
ein Folienträgermaterial (410);
eine erste druckempfindliche Klebeschicht (420); und eine Trennlage (431, 432, 433), die trennbar geschichtet ist, um die erste druckempfindliche Klebeschicht in der genannten Reihenfolge zu bedecken,
wobei:
die Trennlage in ein erstes geteiltes Stück (431), ein zweites geteiltes Stück (432), und ein drittes geteiltes Stück (433) geteilt ist;
das zweite geteilte Stück an einem Ende in Richtung der Breite auf einer Seite der ersten druckempfindlichen Klebeschicht platziert ist, um einen Teil der ersten druckempfindlichen Klebeschicht zu bedecken;
das dritte geteilte Stück an einem Ende in Richtung der Breite auf der anderen Seite der ersten druckempfindlichen Klebeschicht platziert ist, um einen Teil der ersten druckempfindlichen Klebeschicht zu bedecken;
das erste geteilte Stück zwischen dem zweiten geteilten Stück und dem dritten geteilten Stück in Richtung der Breite platziert ist, um einen Bereich der ersten druckempfindlichen Klebeschicht zu bedecken, der nicht mit dem zweiten geteilten Stück und dem dritten geteilten Stück bedeckt ist, wobei das erste geteilte Stück einen erweiternden Bereich (431 b, 431 b') des ersten geteilten Stücks hat, der über das zweite geteilte Stück und das dritte geteilte Stück hinaus erweitert ist; und
das zweite geteilte Stück und das dritte geteilte Stück Faltbereiche (432a, 433a) haben, die zu den Enden in Richtung der Breite hin erweitert sind, und
der Faltbereich (433a) des dritten geteilten Stücks einen erweiternden Bereich des dritten geteilten Stücks (433b) hat, der über das Folienträgermaterial und darüber hinaus erweitert ist.

3. Medizinisches druckempfindliches Heftpflaster nach Anspruch 1, ferner umfassend:
eine Stütze (940); und
eine zweite druckempfindliche Klebeschicht,
wobei:
die Stütze auf einer Seite des Folienträgermaterials gegenüber der ersten druckempfindlichen Klebeschicht platziert ist; und
die zweite druckempfindliche Klebeschicht zwischen der Stütze und dem Folienträgermaterial platziert ist.

4. Medizinisches druckempfindliches Heftpflaster nach Anspruch 3, wobei die Stütze einen Fensterbereich (960) hat.

5. Medizinisches druckempfindliches Heftpflaster nach Anspruch 1, ferner umfassend:
eine Stütze, die einen Fensterbereich hat; und
eine zweite druckempfindliche Klebeschicht, wobei:
die Stütze, die den Fensterbereich hat, zwischen dem Folienträgermaterial und der ersten druckempfindlichen Klebeschicht platziert ist; und
die zweite druckempfindliche Klebeschicht zwischen dem Folienträgermaterial und der Stütze, die den Fensterbereich hat, platziert ist.

6. Folienverband, der unter Verwendung des medizinischen druckempfindlichen Heftpflasters nach Anspruch 1 produziert ist.

7. Bandage, die unter Verwendung des medizinischen druckempfindlichen Heftpflasters nach Anspruch 1 produziert ist.

8. Rezeptur für ein transdermales Absorptionspflaster, die die unter Verwendung des medizinischen druckempfindlichen Heftpflasters nach Anspruch 1 produziert ist.

## Revendications

1. Bande adhésive autocollante médicale (300, 900), comprenant :
un matériau de base en film (310) ;
une première couche adhésive autocollante (320) ; et
une doublure de libération (311, 332, 931, 932) stratifiée de façon libérable pour couvrir la première couche adhésive autocollante dans l'ordre énoncé,
dans laquelle :
la doublure libérable est divisée en une première pièce divisée (331, 931) et une deuxième pièce divisée (332, 932) ;
la première pièce divisée est placée à une extrémité dans la direction de la largeur sur un côté de la première couche adhésive autocollante pour couvrir une partie de la première couche adhésive autocollante, la première pièce divisée ayant une portion d'extension (331b, 931b) de la première pièce divisée s'étendant au-delà de la deuxième pièce divisée ;
la deuxième pièce divisée est placée pour couvrir une portion de la première couche adhésive autocollante qui n'est pas couverte par la première pièce divisée ; et
la deuxième pièce divisée comporte une portion de pliage (332a) s'étendant vers une extrémité dans la direction de la largeur, et la portion de pliage de la deuxième pièce divisée a une portion d'extension de la deuxième pièce divisée (332b) s'étendant sur et au-delà du matériau de base en film.

2. Bande adhésive autocollante médicale (400), comprenant :
un matériau de base en film (410) ;
une première couche adhésive autocollante (420) ; et
une doublure de libération (431, 432, 433) stratifiée de façon libérable pour couvrir la première couche adhésive autocollante dans l'ordre énoncé,
dans laquelle :
la doublure libérable est divisée en une première pièce divisée (431), une deuxième pièce divisée (432) et une troisième pièce divisée (433) ;
la deuxième pièce divisée est placée à une extrémité dans la direction de la largeur sur un côté de la première couche adhésive autocollante pour couvrir une partie de la première couche adhésive autocollante ;
la troisième pièce divisée est placée à une extrémité dans la direction de la largeur sur l'autre côté de la première couche adhésive autocollante pour couvrir une partie de la première couche adhésive autocollante ;
la première pièce divisée est placée entre la deuxième pièce divisée et la troisième pièce divisée dans une direction de la largeur pour couvrir une portion de la première couche adhésive autocollante qui n'est pas couverte par la deuxième pièce divisée et la troisième pièce divisée, la première pièce divisée ayant une portion d'extension (431b, 431b') de la première pièce divisée s'étendant au-delà de la deuxième pièce divisée et de la troisième pièce divisée ; et
la deuxième pièce divisée et la troisième pièce divisée comportant des portions de pliage (432a, 433a) s'étendant vers les extrémités dans la direction de la largeur, et
la portion de pliage (433a) de la troisième pièce divisée a une portion d'extension de la troisième pièce divisée (433b) s'étendant sur et au-delà du matériau de base en film.

3. Bande adhésive autocollante médicale selon la revendication 1, comprenant en outre :
un support (940) ; et
une seconde couche adhésive autocollante,
dans laquelle :
le support est placé sur un côté du matériau de base en film opposé à la première couche adhésive autocollante ; et
la seconde couche adhésive autocollante est placée entre le support et le matériau de base en film.

4. Bande adhésive autocollante médicale selon la revendication 3, dans laquelle le support comporte une portion formant fenêtre (960).

5. Bande adhésive autocollante médicale selon la revendication 1, comprenant en outre :
un support ayant une portion formant fenêtre ; et
une seconde couche adhésive autocollante,
dans laquelle :
le support ayant la portion formant fenêtre est placé entre le matériau de base en film et la première couche adhésive autocollante ; et
la seconde couche adhésive autocollante est placée entre le matériau de base en film et le support ayant la portion formant fenêtre.

6. Film de pansement, qui est produit par l'utilisation de la bande adhésive autocollante médicale selon la revendication 1.

7. Bandage, qui est produit par l'utilisation de la bande adhésive autocollante médicale selon la revendication 1.

8. Formulation de bande d'absorption transdermique, qui est produite par l'utilisation de la bande adhésive autocollante médicale selon la revendication 1.
